# EUROPEAN PATENT APPLICATION

(11) **EP 4 289 340 A1**
(43) Date of publication of application: **13.12.2023**
(21) Application number: 21925531.2
(22) Date of filing: 31.12.2021
(51) Int. Cl.: A61B 5/00

(54) **METHOD AND APPARATUS FOR MEASURING TISSUE COMPOSITION, AND WEARABLE DEVICE**

(30) Priority: 11.02.2021 CN 202110185767
(71) Applicant: Sunrise Technologies Co., Ltd., Fengtai District Beijing 100071 (CN)
(72) Inventor: XU, Kexin, Beijing 100071 (CN); HAN, Tongshuai, Beijing 100071 (CN); ZHAO, Picheng, Beijing 100071 (CN); YAO, Mingfei, Beijing 100071 (CN)
(74) Representative: Isarpatent
(86) International application number: PCT/CN2021/143794
(87) International publication number: WO 2022/170886

(57) **Abstract**

A method of measuring a tissue element, a device (1400) of measuring a tissue element, and a wearable apparatus (2700) are provided. The method includes: determining a positioning feature (S110); determining a measurement region according to the positioning feature, where the measurement region meets a reproducibility of a measurement condition (S120); arranging the measurement probe (1405) at a position corresponding to the measurement region (S130); and performing a tissue element measurement by using the measurement probe (1405) (S140).

## Description

### TECHNICAL FIELD

Embodiments of the present disclosure relate to a field of spectrum measurement technology, and in particular, to a method of measuring a tissue element, a device of measuring a tissue element, and a wearable apparatus.

### BACKGROUND

A variety of tissue elements, such as blood glucose, fat, and white blood cells, etc. are contained in a body fluid of a human body. A concentration of each tissue element is required to be within a corresponding concentration range to ensure a healthy operation of the human body. However, for some individuals, the tissue element is prone to imbalance, that is, the concentration of the tissue element is not within a numerical range, which may lead to diseases, and endanger health and even life. For such objects, there is a need to perform a real-time measurement on the tissue element. During a tissue element measurement process, in order to ensure a measurement accuracy, it is needed to ensure a reproducibility of a measurement condition.

In a process of implementing concepts of the present disclosure, the inventors found that the related art has at least a problem that it is difficult to achieve the reproducibility of the measurement condition by using the related art.

### SUMMARY

In view of this, embodiments of the present disclosure provide a method of measuring a tissue element, a device of measuring a tissue element, and a wearable apparatus.

In an aspect of embodiments of the present disclosure, a method of measuring a tissue element is provided, including: determining a positioning feature; determining a measurement region according to the positioning feature, where the measurement region meets a reproducibility of a measurement condition; arranging the measurement probe at a position corresponding to the measurement region; and performing a tissue element measurement by using the measurement probe.

In another aspect of embodiments of the present disclosure, a device of measuring a tissue element is provided, including: a first determination module configured to determine a positioning feature; a second determination module configured to determine a measurement region according to the positioning feature, where the measurement region meets a reproducibility of a measurement condition; an arrangement module configured to arrange a measurement probe at a position corresponding to the measurement region; and a measurement module configured to perform a tissue element measurement by using the measurement probe.

In another aspect of embodiments of the present disclosure, a wearable apparatus is provided, and the apparatus includes the device of measuring the tissue element as described above.

### BRIEF DESCRIPTION OF THE DRAWINGS

The above and other objectives, features and advantages of the present disclosure will be more apparent through the following description of embodiments of the present disclosure with reference to the accompanying drawings, in which:
FIG. 1 schematically shows a flowchart of a method of measuring a tissue element according to embodiments of the present disclosure;
FIG. 2 schematically shows a schematic diagram of positioning a measurement region based on an optical method according to embodiments of the present disclosure;
FIG. 3 schematically shows another schematic diagram of positioning a measurement region based on an optical method according to embodiments of the present disclosure;
FIG. 4 schematically shows a schematic diagram of positioning a measurement region based on an image matching method according to embodiments of the present disclosure;
FIG. 5 schematically shows another schematic diagram of positioning a measurement region based on an image matching method according to embodiments of the present disclosure;
FIG. 6 schematically shows a schematic diagram of positioning a measurement region based on an imaging method according to embodiments of the present disclosure;
FIG. 7 schematically shows another schematic diagram of positioning a measurement region based on an imaging method according to embodiments of the present disclosure;
FIG. 8 schematically shows a schematic diagram of positioning a measurement posture based on an optical method according to embodiments of the present disclosure;
FIG. 9 schematically shows a schematic diagram of positioning a measurement posture based on an image matching method according to embodiments of the present disclosure;
FIG. 10 schematically shows a schematic diagram of positioning a measurement posture based on an imaging method according to embodiments of the present disclosure;
FIG. 11 schematically shows a schematic diagram of receiving exit light using a photosensitive surface with a small area when a jitter occurs according to embodiments of the present disclosure;
FIG. 12 schematically shows a schematic diagram of receiving exit light using a photosensitive surface with a large area when a jitter occurs according to embodiments of the present disclosure;
FIG. 13 schematically shows a schematic diagram of a measurement result obtained based on a Monte Carlo simulation method according to embodiments of the present disclosure;
FIG. 14 schematically shows a block diagram of a device of measuring a tissue element according to embodiments of the present disclosure;
FIG. 15 schematically shows a schematic diagram of a positional relationship between a measurement probe and a fixing portion according to embodiments of the present disclosure;
FIG. 16 schematically shows a schematic diagram of a fixing portion according to embodiments of the present disclosure;
FIG. 17 schematically shows a schematic diagram of a first fitting part according to embodiments of the present disclosure;
FIG. 18 schematically shows a schematic diagram of another first fitting part according to embodiments of the present disclosure;
FIG. 19 schematically shows a schematic diagram of a region positioning portion according to embodiments of the present disclosure;
FIG. 20 schematically shows a schematic diagram of another region positioning portion according to embodiments of the present disclosure;
FIG. 21 schematically shows a schematic diagram of a first image acquisition portion according to embodiments of the present disclosure;
FIG. 22 schematically shows a schematic diagram of a first posture positioning portion according to embodiments of the present disclosure;
FIG. 23 schematically shows a schematic diagram of another first posture positioning portion according to embodiments of the present disclosure;
FIG. 24 schematically shows a schematic diagram of a third image acquisition portion according to embodiments of the present disclosure;
FIG. 25 schematically shows a schematic diagram of positioning a measurement posture and a measurement region according to embodiments of the present disclosure;
FIG. 26 schematically shows another schematic diagram of positioning a measurement posture and a measurement region according to embodiments of the present disclosure;
FIG. 27 schematically shows a schematic diagram of a wearable apparatus according to embodiments of the present disclosure;
FIG. 28 schematically shows a schematic diagram of an assembly process of a wearable apparatus according to embodiments of the present disclosure;
FIG. 29 schematically shows a schematic diagram of maintaining an average optical path of the exit light received by the measurement probe within a predetermined optical path range during a skin jitter process in a case that the wearable apparatus is consistent with a skin jitter pattern according to embodiments of the present disclosure; and
FIG. 30 schematically shows a schematic diagram of maintaining an average optical path of the exit light received by the measurement probe within a predetermined optical path range during a skin jitter process in a case that the wearable apparatus causes a movement amplitude of a skin at the measurement region to be less than or equal to a movement amplitude threshold according to embodiments of the present disclosure.

### DETAILED DESCRIPTION OF EMBODIMENTS

Embodiments of the present disclosure will be described below with reference to the accompanying drawings. It should be understood, however, that these descriptions are merely exemplary and are not intended to limit the scope of the present disclosure. In the following detailed descriptions, for ease of interpretation, many specific details are set forth to provide a comprehensive understanding of embodiments of the present disclosure. However, it is clear that one or more embodiments may also be implemented without these specific details. In addition, in the following descriptions, descriptions of well-known structures and technologies are omitted to avoid unnecessarily obscuring concepts of the present disclosure.

Terms used herein are for the purpose of describing specific embodiments only and are not intended to limit the present disclosure. The terms "including", "containing", etc. used herein indicate the presence of the feature, step, operation and/or component, but do not exclude the presence or addition of one or more other features, steps, operations or components.

All terms used herein (including technical and scientific terms) have the meanings generally understood by those skilled in the art, unless otherwise defined. It should be noted that the terms used herein shall be interpreted to have meanings consistent with the context of this specification, and shall not be interpreted in an idealized or overly rigid manner.

In a case of using the expression similar to "at least one selected from A, B, and C", it should be explained according to the meaning of the expression generally understood by those skilled in the art (for example, "a system including at least one selected from A, B, and C" should include but not be limited to a system including A alone, a system including B alone, a system including C alone, a system including A and B, a system including A and C, a system including B and C, and/or a system including A, B and C). In a case of using the expression similar to "at least one selected from A, B, or C", it should be explained according to the meaning of the expression generally understood by those skilled in the art (for example, "a system including at least one selected from A, B, or C" should include but not be limited to a system including A alone, a system including B alone, a system including C alone, a system including A and B, a system including A and C, a system including B and C, and/or a system including A, B and C).

A research on a living tissue element measurement based on an optical method has experienced nearly fifty years of development, and a large number of scientific research institutes and companies have invested great research enthusiasm in this field. Due to a weak absorption of a measured tissue element and a small variation range of a concentration of the measured tissue element of the measured object, a signal of the measured tissue element is generally weak, and the weak signal of the measured tissue element may be easily submerged by an interference such as a change in a measurement condition. Up to now, there has not appeared a solution that may achieve a reliable living tissue element measurement. Therefore, the living tissue element measurement is a global problem that needs to be solved. The tissue element may include blood glucose, fat, and white blood cells, etc. The signal of the measured tissue element represents a change in an output light intensity caused by a change in a concentration of the measured tissue element. The measurement condition may be understood as a condition that affects a transmission path of light in a tissue. The measurement condition may include a controllable measurement condition and an uncontrollable measurement condition. The controllable measurement condition refers to a measurement condition that may be controlled within a predetermined change range (that is, kept unchanged or substantially unchanged) by adopting an effective control method during each tissue element measurement process. The uncontrollable measurement condition refers to a measurement condition with unpredictable and uncontrollable characteristics. The controllable measurement condition may include a temperature, a pressure, a measurement region and a measurement posture, etc. The uncontrollable measurement condition may include a change in a physiological background and a drift of a measurement device, etc.

For a control of the controllable measurement condition, the inventors found that the changes in the controllable measurement conditions affect the measurement results through different mechanisms, and it is difficult to suppress the influence on the measurement result by using a mathematical algorithm, but the reproducibility of the controllable measurement condition may be achieved using an effective control method, so that the influence of the controllable measurement condition on the measurement result may be reduced to a negligible level, that is, the influence of the change in the controllable measurement condition on the measurement result is at a similar level to an influence of a random noise on the measurement result. Therefore, for the processing of the controllable measurement condition, the inventors propose that an appropriate processing method is to control the controllable measurement condition to achieve the reproducibility of the controllable measurement condition by using an effective control method. The effective control method may be implemented with a hardware design. The reproducibility of the controllable measurement condition may refer to that during each tissue element measurement, the controllable measurement condition is maintained within a predetermined change range, so that the controllable measurement condition remains unchanged or substantially unchanged.

Embodiments of the present disclosure mainly focus on a reproducibility of a measurement posture and a reproducibility of a measurement region. The measurement posture refers to a posture of a limb supporting a measurement site. In the related art, no relevant content was found for the measurement posture.

First, for the reproducibility of the measurement region, a positioning deviation of the measurement region is caused by a non-uniformity of a tissue distribution and a difference in a flatness of a skin surface. When a relative position between the measurement probe and the measurement region deviates, the transmission path of light in the tissue may be changed. Therefore, in order to achieve the reproducibility of the controllable measurement condition, the reproducibility of the measurement region needs to be ensured as much as possible.

Second, for the reproducibility of the measurement posture, it is difficult for the measured object to keep a same measurement posture during the tissue element measurement, and a change in the measurement posture may cause a change in the skin state of the measurement region, which may lead to a change in the transmission path of light in the tissue. Therefore, the change in the measurement posture may cause a positioning deviation and then affect a reliability of the measurement result. The skin state may include a surface shape of the skin and an internal structure of the skin. Therefore, it is necessary to achieve the reproducibility of the measurement posture. A purpose of positioning the measurement posture is to ensure that the measurement posture is a target measurement posture when a tissue element measurement is performed, that is, when the tissue element measurement is performed, if a current measurement posture is not the target measurement posture, it is needed to adjust the current measurement posture to the target measurement posture. The target measurement posture is a measurement posture that meets the reproducibility of the controllable measurement condition.

However, actually, an importance of achieving the reproducibility of the measurement posture is often overlooked, which is reflected in the following two aspects.

In a first aspect, it was not found that the reproducibility of the measurement posture is an important factor affecting the obtaining of a true signal of the measured tissue element. In the related art, it is generally considered that in terms of the controllable measurement condition, a main factor affecting the reproducibility of the controllable measurement condition is the reproducibility of the measurement region, that is, it is considered that an improvement of a possibility of obtaining a true signal of the measured tissue element may be achieved in terms of the controllable measurement condition if the reproducibility of the measurement region is achieved, and that it is not needed to consider other factors. In other words, in the related art, a direction of the improvement revolves around how to improve a positioning accuracy of the measurement region, and it has not been found that in terms of the controllable measurement condition, the reproducibility of the measurement posture is also an important factor affecting the possibility of obtaining the true signal of the measured tissue element.

Moreover, according to the above analysis, even if the reproducibility of the measurement region is achieved, when the posture of the limb supporting the measurement region changes, the internal structure of the skin at the measurement region may also change, which results in a change in the transmission path of light in the tissue, thereby affecting the possibility of obtaining the true signal of the measured tissue element. In other words, only ensuring the reproducibility of the measurement region while ignoring the reproducibility of the measurement posture is not conducive to an improvement of the possibility of obtaining the true signal of the measured tissue element.

In a second aspect, no effective method is adopted to achieve the reproducibility of the measurement posture. Due to the lack of deep research on the factors affecting the obtaining of the true signal of the measured tissue element, the importance of achieving the reproducibility of the measurement posture has not been recognized. In the tissue element measurement, it is considered that the method of keeping a body stability by the measured object is sufficient to achieve a control of the measurement posture, that is, the measurement posture is well controlled if the measured object believes that a body state is not changed. However, in most cases, the change in the measurement posture may not be perceived by the measured object, and such method of achieving the reproducibility of the measurement posture may result in a significant error, which may greatly interfere with the measurement result. Even if a method is adopted to control the measurement posture, such method substantially may not ensure the reproducibility of the measurement posture since the importance of achieving the reproducibility of the measurement posture is not recognized.

Therefore, in order to achieve the reproducibility of the measurement region, it is needed to ensure the reproducibility of the measurement posture as much as possible, that is, to accurately position the measurement posture. Based on the above, the reproducibility of the measurement region needs to be based on the reproducibility of the measurement posture. Therefore, the positioning of the measurement region needs to be based on the positioning of the measurement posture.

Following this way, embodiments of the present disclosure provide a solution for a tissue element measurement, including: determining a measurement region according to a positioning feature, where the measurement region is a region that meets a reproducibility of a measurement condition; arranging a measurement probe at a position corresponding to the measurement region; and performing a tissue element measurement by using the measurement probe. The positioning feature is used to positioning the measurement posture and/or the measurement region. A description will be given below in conjunction with specific embodiments.

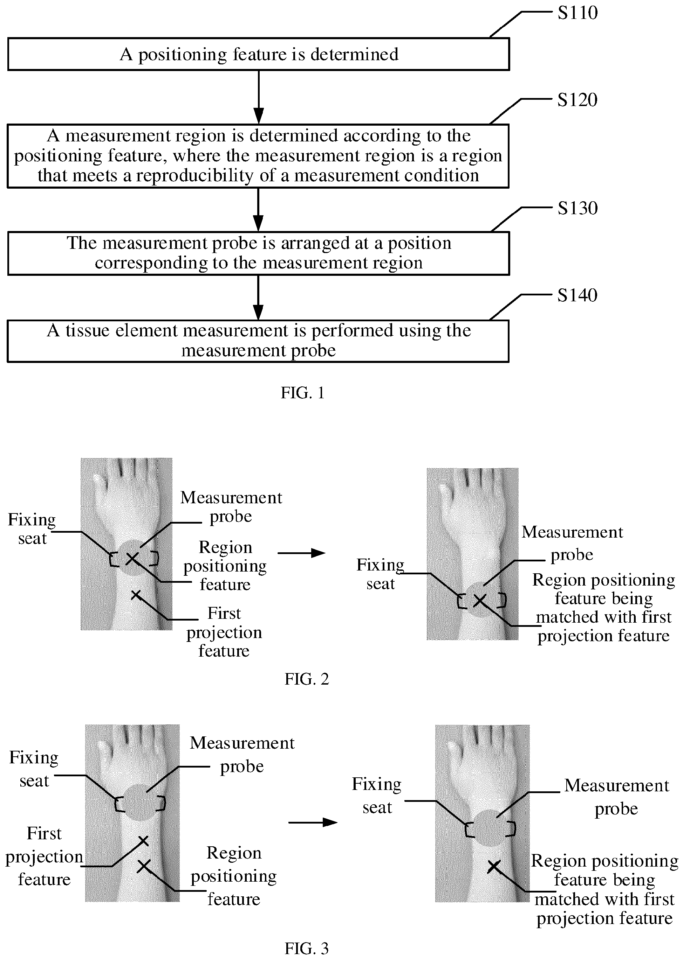

FIG. 1 schematically shows a flowchart of a method of measuring a tissue element according to embodiments of the present disclosure.

As shown in FIG. 1, the method includes operations S 110 to S140.

In operation S 110, a positioning feature is determined.

According to embodiments of the present disclosure, during a positioning process, the positioning may be performed according to a positioning feature. The positioning feature may include a posture positioning feature and a region positioning feature. The posture positioning feature is used for the positioning of the measurement posture, and the region positioning feature is used for the positioning of the measurement region. The posture positioning feature may be arranged on a measured object or a non-measured object, and the region positioning feature may be arranged on the measured object or the non-measured object. The non-measured object may include the measurement probe or other devices. The positioning feature may include a manually provided positioning feature or an inherent feature of the measured object. The inherent feature on the measured object may include palm print, fingerprint, birthmark, mole or nevus, etc.

According to embodiments of the present disclosure, if a method of manually providing a positioning feature is adopted, the manually provided positioning feature may gradually fade over time, and it is required to provide the positioning feature again, which may introduce a new error and affect the positioning accuracy. The inherent feature on the measured object has a good stability and is not prone to an arrangement error.

The inherent feature on the measured object may be used as the positioning feature in order to reduce a positioning complexity and improve a positioning accuracy. However, even if the inherent feature on the measured object is used as the posture positioning feature, the internal structure of the skin may be affected by the change in the measurement posture, which may also result in a positioning deviation of the measurement region. Therefore, the position where the positioning feature is arranged on the measured object may not be determined arbitrarily, but according to the measurement site and a bone-muscle relationship between the measurement site and a peripheral site. Exemplarily, if the measurement site is a forearm extensor side, the peripheral site includes a wrist. For the forearm extensor side, a change in a state of the wrist may greatly affect the skin state of the forearm extensor side. In order to improve the positioning accuracy, the positioning features may be arranged on the forearm extensor side and a back of a hand respectively. It should be noted that the positioning feature may be manually provided if no inherent feature on the measured object may be used as the positioning feature. For example, the positioning feature may be a point marker or a graphic marker, and the graphic marker may include a cross marker.

In operation S 120, a measurement region is determined according to the positioning feature, where the measurement region is a region that meets a reproducibility of a measurement condition.

In operation S130, the measurement probe is arranged at a position corresponding to the measurement region.

In operation S140, a tissue element measurement is performed using the measurement probe.

According to embodiments of the present disclosure, the measurement region determined based on the positioning feature is the region that meets the reproducibility of the measurement condition. Performing the tissue element measurement by using the measurement probe arranged at the position corresponding to the measurement region includes: irradiating the measurement region by incident light having at least one predetermined wavelength, where each beam of the incident light is incident on an incident position to form at least one beam of exit light exited from at least one exit position on the measurement region; obtaining a light intensity value corresponding to each beam of exit light acquired by the measurement probe, so as to obtain T output light intensities, where the measurement probe includes M photosensitive surfaces, and each output light intensity is obtained by processing the light intensity value of the exit light acquired by one or more photosensitive surfaces; and determining a concentration of the measured tissue element according to at least one output light intensity corresponding to the at least one predetermined wavelength.

According to the technical solutions of embodiments of the present disclosure, by determining the positioning feature, determining the measurement region meeting the reproducibility of the measurement condition according to the positioning feature, arranging the measurement probe at the position corresponding to the measurement region, and performing a tissue element measurement using the measurement probe, the measurement region may be positioned accurately, and then the reproducibility of the measurement condition may be effectively controlled.

According to embodiments of the present disclosure, the positioning feature includes a first posture positioning feature and a region positioning feature. Determining the measurement region according to the positioning feature may include the following operations.

A current measurement posture of the measured object is adjusted to a target measurement posture according to the first posture positioning feature, where the target measurement posture is a measurement posture meeting the reproducibility of the measurement condition. When the current measurement posture is the target measurement posture, the measurement region is determined according to the region positioning feature.

According to embodiments of the present disclosure, when positioning the measurement posture and the measurement region, the positioning of the measurement posture is the basis of the positioning of the measurement region. In a subsequent measurement process after completing the positioning of the measurement region, it is generally not required to reposition the measurement region, but there may be a case that it is still required to position the measurement posture. A condition for completing the positioning of the measurement posture is that the current measurement posture is the target measurement posture. The target measurement posture is a measurement posture that meets the reproducibility of the measurement condition.

According to embodiments of the present disclosure, a cause for the case that it is still required to position the measurement posture lies in that a strategy of allowing the measured site to move within a certain range when no measurement is performed and positioning the measurement posture when a measurement is performed is adopted in embodiments of the present disclosure in order to bring a better user experience to the measured object. When performing a measurement, it is required to ensure that the current measurement posture is the target measurement posture. If the current measurement posture is not the target measurement posture, it is needed to adjust the measurement posture to ensure that the current measurement posture is the target measurement posture.

Based on the above, the positioning may be divided into a first positioning of the measurement posture, a positioning of the measurement region, and a second positioning of the measurement posture. The first positioning of the measurement posture may be understood as a positioning of the measurement posture in cooperation with the implementation of the measurement region. The second positioning of the measurement posture may be understood as a positioning of the measurement posture that is performed in a case that the measurement posture is not the target measurement posture after the measurement probe is arranged at the position corresponding to the measurement region.

According to embodiments of the present disclosure, the region positioning feature is used for the positioning of the measurement region. The posture positioning feature used for the first positioning of the measurement posture is referred to as a first posture positioning feature. The posture positioning feature used for the second positioning of the measurement posture is referred to as a second posture positioning feature. Both the first posture positioning feature and the second posture positioning feature are used for the positioning of the measurement posture. The region positioning feature, the first posture positioning feature and the second posture positioning feature may be different from each other, partially the same, or all the same. There may be one or more region positioning features, first posture positioning features and second posture positioning features.

When performing the first positioning of the measurement posture and the positioning of the measurement region, the current measurement posture of the measured object may be adjusted according to the first posture positioning feature so that the first posture positioning feature is matched with a predetermined feature. When the first posture positioning feature is matched with the predetermined feature, it may be determined that the current measurement posture is the target measurement posture. When the current measurement posture is the target measurement posture, the measurement region is determined according to the region positioning feature. Then, the positioning of the measurement posture and the positioning of the measurement region are completed.

It should be noted that determining the measurement region according to the region positioning feature may be understood as determining a region corresponding to the region positioning feature as the measurement region, which includes determining a region where the region positioning feature is located as the measurement region, or determining another region having an association relationship with the region positioning feature as the measurement region.

By using the first posture positioning feature and the region positioning feature, the positioning of the measurement region and the positioning of the measurement posture may be achieved synchronously.

According to embodiments of the present disclosure, arranging the measurement probe at the position corresponding to the measurement region may include the following operations.

The measurement probe is arranged at the position corresponding to the measurement region by a fixing portion. The fixing portion is integrated with, partially separated from or completely separated from the measurement probe.

According to embodiments of the present disclosure, the fixing portion is used to fix the measurement probe. The fixing portion may be integrated with, partially separated from or completely separated from the measurement probe, that is, the fixing portion may be a component of the measurement probe, or may be independent of the measurement probe, or partially be a component of the measurement probe and partially be independent of the measurement probe. The fixing portion may include a fixing seat and a first fitting part, or the fixing portion may include a second fitting part. The first fitting part is used to arrange the fixing seat at the position corresponding to the measurement region, and the fixing seat is used to arrange the measurement probe. The second fitting part is used to arrange the measurement probe at the position corresponding to the measurement region.

If the fixing portion includes the fixing seat and the first fitting part, the fixing seat is separated from the measurement probe, and the first fitting part is integrated with or separated from the fixing seat. If the fixing portion includes the second fitting part, the second fitting part is integrated with or separated from the measurement probe.

According to embodiments of the present disclosure, the fixing portion includes the fixing seat and the first fitting part.

Arranging the measurement probe at the position corresponding to the measurement region by the fixing portion may include the following operations.

The fixing seat is arranged at the position corresponding to the measurement region by the first fitting part. The measurement probe is arranged on the fixing seat.

According to embodiments of the present disclosure, the measurement probe is arranged at the position corresponding to the measurement region by the fixing seat, rather than directly arranged at the position corresponding to the measurement region.

During the tissue element measurement process, if the measurement probe is arranged at the position corresponding to the measurement region by the fixing seat, the fixing seat may be arranged on the measurement region for a long time without leaving the measurement region, and the measurement probe may be arranged on the fixing seat when a measurement is performed and may be detached from the fixing seat when no measurement is performed. Moreover, since the fixing seat is arranged at the position corresponding to the measurement region, it is possible to maintain a good positioning accuracy and reduce the difficulty of positioning the measurement probe when the measurement probe is detached from the fixing seat and then arranged on the fixing seat.

According to embodiments of the present disclosure, the skin state of the skin at the measurement region meets a first predetermined condition in a process of arranging the fixing seat at the position corresponding to the measurement region by the first fitting part.

According to embodiments of the present disclosure, the skin state of the skin at the measurement region meets a second predetermined condition in a process of arranging the measurement probe on the fixing seat.

According to embodiments of the present disclosure, an action of fixing the fixing seat may affect the skin state of the skin at the corresponding position, and then affect the positioning accuracy of the measurement region. In order to ensure the positioning accuracy of the measurement region, it is possible to ensure that the skin state of the skin at the measurement region meets the first predetermined condition in the process of fixing the fixing seat by the first fitting part. The first predetermined condition may refer to that a change in the skin state of the skin at the corresponding position is within a first predetermined range in the process of fixing the fixing seat by the first fitting part. The change in the skin state may include a skin deformation. Accordingly, the first predetermined range may include a first predetermined deformation range.

According to embodiments of the present disclosure, an action of fixing the measurement probe may affect the skin state of the skin at the corresponding position, and then affect the positioning accuracy of the measurement region. In order to ensure the positioning accuracy of the measurement region, it is possible to ensure that the skin state of the skin at the measurement region meets the second predetermined condition in the process of fixing the measurement probe by the fixing seat. The second predetermined condition may refer to that a change in the skin state of the skin at the corresponding position is within a second predetermined range in the process of fixing the measurement probe by the fixing seat. The change in the skin state may include a skin deformation. Accordingly, the second predetermined range may include a second predetermined deformation range.

According to embodiments of the present disclosure, the measurement probe is non-movable on the fixing seat.

According to embodiments of the present disclosure, when the measurement probe is fixed on the fixing seat, there may be a problem of an unstable fixation that may affect the reproducibility of the measurement condition. In order to solve this problem, it is possible to ensure that the measurement probe does not move on the fixing seat during the tissue element measurement process.

According to embodiments of the present disclosure, the fixing portion includes the second fitting part.

Arranging the measurement probe at the position corresponding to the measurement region by the fixing portion may include the following operations.

The measurement probe is arranged at the position corresponding to the measurement region by the second fitting part.

According to embodiments of the present disclosure, for the method of arranging the measurement probe at the position corresponding to the measurement region, in addition to the above-mentioned method of arranging the measurement probe at the position corresponding to the measurement region by the fixing seat, it is also possible to adopt a method of directly arranging the measurement probe at the position corresponding to the measurement region, in which the fixing seat is not required, but a cooperation of the second fitting part is required.

It should be noted that the above-mentioned not requiring the fixing seat may include the following two cases. In a first case, the measurement probe is provided with a structure integrated with the measurement probe, which plays a same role as an independent fixing seat. In a second case, the measurement probe is not provided with a structure that plays the same role as an independent fixing seat.

According to embodiments of the present disclosure, the skin state of the skin at the measurement region meets a third predetermined condition in a process of arranging the measurement probe at the position corresponding to the measurement region by the second fitting part.

According to embodiments of the present disclosure, an action of fixing the measurement probe may affect the skin state of the skin at the corresponding position, and then affect the positioning accuracy of the measurement region. In order to ensure the positioning accuracy of the measurement region, it is possible to ensure that the skin state of the skin at the measurement region meets the third predetermined condition in the process of fixing the measurement probe by the second fitting part. The third predetermined condition may refer to that a change in the skin state of the skin at the corresponding position is within a third predetermined range in the process of fixing the measurement probe by the second fitting part. The change in the skin state may include a skin deformation. Accordingly, the third predetermined range may include a third predetermined deformation range.

According to embodiments of the present disclosure, determining the measurement region according to the region positioning feature may include the following operations.

A first projection feature is acquired. When it is determined that the region positioning feature is not matched with the first projection feature, a position of the measurement probe and/or the fixing portion is adjusted until the region positioning feature is matched with the first projection feature. When it is determined that the region positioning feature is matched with the first projection feature, a region corresponding to the measurement probe and/or the fixing portion is determined as the measurement region.

According to embodiments of the present disclosure, in order to ensure a flexibility of use and the positioning accuracy of the measurement region, it is possible to adopt an optical method, that is, to match the region positioning feature with the first projection feature, and determine the measurement region according to a matching result. The first projection feature is formed according to the optical method, that is, by projecting a light spot with a predetermined shape using a light source. The shape of the light spot may be determined according to the region positioning feature. For example, the light spot with the predetermined shape is a cross light spot.

According to embodiments of the present disclosure, after the first projection feature is obtained by using a structure for projecting the first projection feature, it is determined whether the region positioning feature is matched with the first projection feature. When it is determined that the region positioning feature is not matched with the first projection feature, the position of the measurement probe and/or the fixing seat may be adjusted so that the region positioning feature is matched with the first projection feature, until the region positioning feature is matched with the first projection feature. When it is determined that the region positioning feature is matched with the first projection feature, it may indicate that the region where the measurement probe and/or the fixing seat are/is currently located is the measurement region.

According to embodiments of the present disclosure, the structure for projecting the first projection feature may be arranged on the measured object, the measurement probe, the fixing seat or other objects. Other objects may refer to objects other than the measurement probe, the fixing portion and the measured object. The region positioning feature may be arranged on at least one selected from the measurement probe, the fixing seat, the measured object, or other objects. An adjustment process implemented based on the optical method will be described below from two aspects, including an arrangement position of the structure for projecting the first projection feature and an arrangement position of the region positioning feature.

A description is given below from the aspect of the arrangement position of the structure for projecting the first projection feature.

First, if the structure for projecting the first projection feature is arranged on the measured object, the region positioning feature may be arranged on at least one selected from the measured object, the measurement probe, the fixing seat, or other objects. It should be noted that if the region positioning feature is arranged on the measured object or other objects, the positioning of the measurement region may be achieved by a method of adjusting the position of the measurement probe and/or the fixing seat according to the region positioning feature and the first projection feature until the region positioning feature is matched with the first projection feature. The region positioning feature being matched with the first projection feature here means that the region positioning feature is blocked by the measurement probe and/or the fixing seat, so that the first projection feature may not be projected to the position where the region positioning feature is located. If the region positioning feature is not matched with the first posture positioning feature, at least one first projection feature may be projected to the position where the region positioning feature is located.

Second, if the structure for projecting the first projection feature is arranged on the measurement probe, the region positioning feature may not be arranged on the measurement probe, but may be arranged on the measured object, the fixing seat or other objects. It should be noted that if the region positioning feature is arranged on the fixing seat, and the positioning of the measurement probe is achieved by arranging the measurement probe at the position corresponding to the measurement region by the fixing portion provided with the fixing seat, the positioning of the measurement region may be achieved by the method of adjusting the position of the fixing seat. The position of the measurement probe is fixed before the region positioning feature is matched with the first projection feature. The position of the fixing seat is adjusted according to the region positioning feature and the first projection feature until the region positioning feature is matched with the first projection feature. When the two are matched, the region corresponding to the fixing seat is determined as the measurement region, and then the measurement probe may be arranged on the fixing seat.

Third, if the structure for projecting the first projection feature is arranged on the fixing seat, the region positioning feature may not be arranged on the fixing seat, but may be arranged on the measured object, the measurement probe or other objects. It should be noted that if the region positioning feature is arranged on the measurement probe, and the positioning of the measurement probe is achieved by arranging the measurement probe at the position corresponding to the measurement region by the fixing portion provided with the fixing seat, then the positioning of the measurement region may be achieved by the method of adjusting the position of the fixing seat. The position of the measurement probe is fixed before the region positioning feature is matched with the first projection feature. The position of the fixing seat is adjusted according to the region positioning feature and the first projection feature until the region positioning feature is matched with the first projection feature. When the two are matched, the region corresponding to the fixing seat is determined as the measurement region, and then the measurement probe may be arranged on the fixing seat.

Fourth, if the structure for projecting the first projection feature is arranged on other objects, the region positioning feature may be arranged on at least one selected from the measured object, the measurement probe, the fixing seat, or other objects. It should be noted that if the region positioning feature is arranged on the measured object or other objects, the positioning of the measurement region may be achieved using a method similar to that of arranging the structure for projecting the first projection feature on the measured object and arranging the region positioning feature on the measured object or other objects, which will not be repeated here.

A description will be given below from the aspect of the arrangement position of the region positioning feature.

First, if the region positioning feature is arranged on the measured object, the structure for projecting the first projection feature may be arranged on the measured object, the measurement probe, the fixing seat or other objects. It should be noted that if the structure for projecting the first projection feature is arranged on the measured object or other objects, the positioning of the measurement region may be achieved by the method of adjusting the position of the measurement probe and /or the fixing seat according to the region positioning feature and the first projection feature until the region positioning feature is matched with the first projection feature. The region positioning feature being matched with the first projection feature here means that the region positioning feature is blocked by the measurement probe and/or the fixing seat, so that the first projection feature may not be projected to the position where the region positioning feature is located. If the region positioning feature is not matched with the first posture positioning feature, at least one first projection feature may be projected to the position where the region positioning feature is located.

Second, if the region positioning feature is arranged on the measurement probe, the structure for projecting the first projection feature is separated from the measurement probe, and may be arranged on the measured object, the fixing seat or other objects. It should be noted that if the structure for projecting the first projection feature is arranged on the fixing seat, reference may be made to the above description of the corresponding part, and details will not be repeated here.

Third, if the region positioning feature is arranged on the fixing seat, the structure for projecting the first projection feature is separated from the fixing seat, and may be arranged on the measured object, the measurement probe or other objects. It should be noted that if the structure for projecting the first projection feature is arranged on the measurement probe, reference may be made to the above description of the corresponding part, and details will not be repeated here.

Fourth, if the region positioning feature is arranged on other objects, the structure for projecting the first projection feature may be arranged on the measured object, the measurement probe, the fixing seat or other objects. It should be noted that if the structure for projecting the first projection feature is arranged on the measured object or other objects, reference may be made to the above description of the corresponding part, and details will not be repeated here.

Exemplarily, FIG. 2 schematically shows a schematic diagram of positioning the measurement region based on an optical method according to embodiments of the present disclosure. In FIG. 2, the region positioning feature is arranged on the measurement probe. FIG. 3 schematically shows another schematic diagram of positioning the measurement region based on an optical method according to embodiments of the present disclosure. In FIG. 3, the region positioning feature is arranged on the measured object.

When positioning the measurement region using an optical method, on the one hand, a position and an angle of the light source may be adjusted flexibly and may be easily matched with the region positioning feature. Therefore, the region positioning feature may be arranged flexibly, so that the difficulty in the arrangement of the region positioning feature is reduced. On the other hand, the shape of the exit light spot may be adjusted to be matched with the region positioning feature better, so that the positioning accuracy may be improved.

According to embodiments of the present disclosure, determining the measurement region according to the region positioning feature may include the following operations.

A first target image is obtained. A first template image is obtained, where the first template image contains the region positioning feature. When it is determined that the first target image is not matched with the first template image, the position of the measurement probe and/or the fixing portion is adjusted to obtain a new first target image until the new first target image is matched with the first template image. When it is determined that the first target image is matched with the first template image, the region corresponding to the measurement probe and/or the fixing portion is determined as the measurement region.

According to embodiments of the present disclosure, in order to ensure the flexibility of use and the positioning accuracy of the measurement region, it is possible to adopt an image matching method, that is, to match the first target image with the first template image, and determine the measurement region according to a matching result. The first template image may contain the region positioning feature, and a position of the region positioning feature in the first template image is a predetermined position. In the process of matching the first target image with the first template image, the first target image may be a target image that does not contain the region positioning feature, or may be a target image that contains the region positioning feature but the position of the region positioning feature in the first target image is not the predetermined position, or may be a target image that contains the region positioning feature and the position of the region positioning feature in the first target image is the predetermined position. Since the first template image contains the region positioning feature at the predetermined position, if the first target image is matched with the first template image, it may indicate that the first target image contains the region positioning feature and the position of the region positioning feature in the first target image is the predetermined position. In other words, a purpose of matching the first target image with the first template image is to obtain such first target image that the region positioning feature is contained in the first target image and the position of the region positioning feature in the first target image is the predetermined position.

According to embodiments of the present disclosure, when it is determined that the first target image is matched with the first template image, it may indicate that the region where the measurement probe and/or the fixing seat are/is currently located is the measurement region. Determining whether the first target image is matched with the first template image may include determining a similarity between the first target image and the first template image. If the similarity is greater than or equal to a similarity threshold, it is determined that the first target image is matched with the first template image. If the similarity is less than the similarity threshold, it is determined that the first target image is not matched with the first template image. Determining the similarity between the first target image and the first template image may include performing a correlation analysis on the first target image and the first template image to obtain a correlation coefficient, and determining the similarity between the first target image and the first template image according to the correlation coefficient.

According to embodiments of the present disclosure, a structure for acquiring the first target image may be arranged on the measured object, the measurement probe, the fixing seat or other objects. Other objects may refer to objects other than the measurement probe, the fixing portion and the measured object. The region positioning feature may be arranged on at least one selected from the measurement probe, the fixing seat, the measured object, or other objects. For the description of the region positioning feature and the structure for acquiring the first target image, reference may be made to the description of the region positioning feature and the structure for projecting the first projection feature, and details will not be repeated here. A difference is that the region positioning feature may be arranged on at least one selected from the measured object, the measurement probe, the fixing seat, or other objects if the structure for acquiring the first target image is arranged on the measurement probe; the region positioning feature may be arranged on at least one selected from the measured object, the measurement probe, the fixing seat, or other objects if the structure for acquiring the first target image is arranged on the fixing seat.

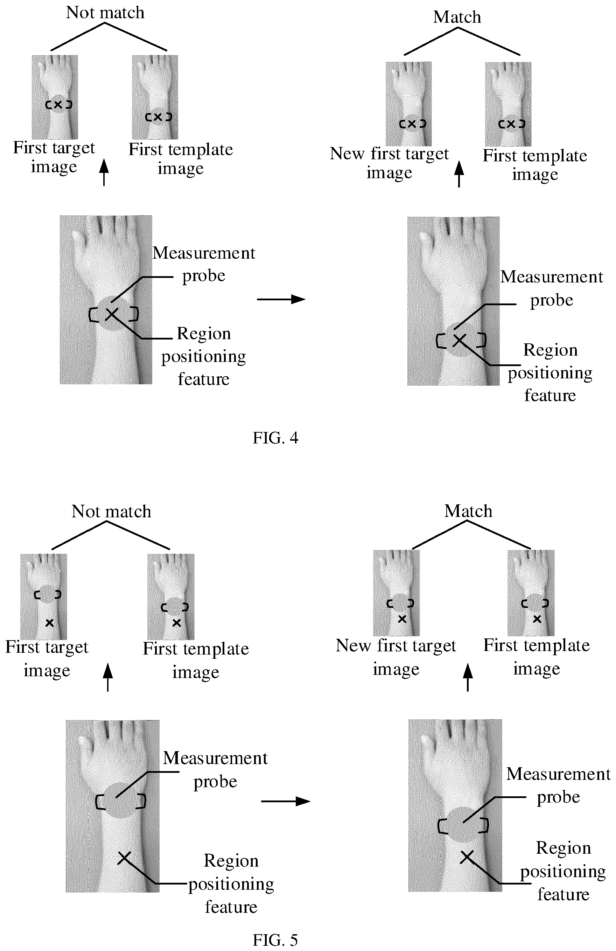

Exemplarily, FIG. 4 schematically shows a schematic diagram of positioning the measurement region based on an image matching method according to embodiments of the present disclosure. In FIG. 4, the region positioning feature is arranged on the measurement probe. FIG. 5 schematically shows another schematic diagram of positioning the measurement region based on an image matching method according to embodiments of the present disclosure. In FIG. 5, the region positioning feature is arranged on the measured object.

According to embodiments of the present disclosure, determining the measurement region according to the region positioning feature may include the following operations.

A second target image is obtained, where the second target image contains the region positioning feature. When it is determined that the position of the region positioning feature in the second target image is not the first predetermined position, the position of the measurement probe and/or the fixing portion is adjusted to obtain a new second target image until the position of the region positioning feature in the new second target image is the first predetermined position. When it is determined that the position of the region positioning feature in the new second target image is the first predetermined position, the region corresponding to the measurement probe and/or the fixing portion is determined as the measurement region.

According to embodiments of the present disclosure, in order to ensure the flexibility of use and the positioning accuracy of the measurement region, it is possible to adopt an imaging method, that is, if the position of the region positioning feature in the second target image is the first predetermined position, it may indicate that the positioning of the measurement region is completed.

According to embodiments of the present disclosure, the process of positioning the measurement region using the imaging method is a process of determining whether the position of the region positioning feature in the second target image is the first predetermined position. If the position of the region positioning feature in the second target image is not the first predetermined position, the position of the measurement probe and/or the fixing seat may be adjusted to obtain a new second target image until the position of the region positioning feature in the new second target image is the first predetermined position. When the position of the region positioning feature in the new second target image is the first predetermined position, it may indicate that the region where the measurement probe and/or the fixing seat are/is currently located is the measurement region.

According to embodiments of the present disclosure, a structure for acquiring the second target image may be arranged on the measured object, the measurement probe, the fixing seat or other objects. Other objects may refer to objects other than the measurement probe, the fixing portion and the measured object. The region positioning feature may be arranged on at least one selected from the measurement probe, the fixing seat, the measured object, or other objects. For the description of the region positioning feature and the structure for acquiring the second target image, reference may be made to the description of the region positioning feature and the structure for projecting the first projection feature, and details will not be repeated here.

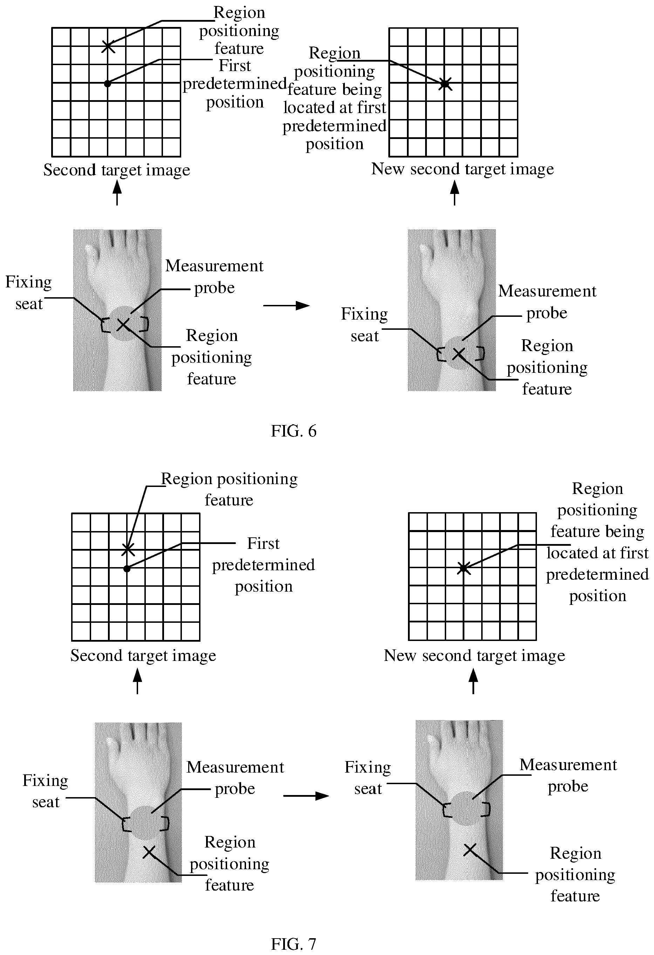

Exemplarily, FIG. 6 schematically shows a schematic diagram of positioning the measurement region based on an imaging method according to embodiments of the present disclosure. In FIG. 6, the region positioning feature is arranged on the measurement probe. FIG. 7 schematically shows another schematic diagram of positioning the measurement region based on an imaging method according to embodiments of the present disclosure. In FIG. 7, the region positioning feature is arranged on the measured object. In FIG. 7, a movement of the measurement probe and the fixing seat causes a change in a relative position between the two and the region positioning feature, so that the position of the region positioning feature presented in the image is the first predetermined position.

According to embodiments of the present disclosure, adjusting the current measurement posture of the measured object to the target measurement posture according to the first posture positioning feature may include the following operations.

A second projection feature is obtained. When it is determined that the first posture positioning feature is not matched with the second projection feature, the current measurement posture is adjusted until the first posture positioning feature is matched with the second projection feature. When it is determined that the first posture positioning feature is matched with the second projection feature, it is determined that the current measurement posture is the target measurement posture.

According to embodiments of the present disclosure, in order to ensure the flexibility of use and the positioning accuracy of the measurement posture, it is possible to adopt an optical method, that is, to match the first posture positioning feature with the second projection feature, and determine the target measurement posture according to a matching result. The second projection feature is formed according to the optical method, that is, by projecting a light spot with a predetermined shape using a light source. The shape of the light spot may be determined according to the first posture positioning feature. That is, for the measured object, the second projection feature matched with the first posture positioning feature is arranged according to the first posture positioning feature. The current measurement posture with which the first posture positioning feature is matched with the second projection feature is the target measurement posture.

According to embodiments of the present disclosure, the structure for projecting the second projection feature may be arranged on the measured object, the measurement probe, the fixing seat or other objects. Other objects may refer to objects other than the measurement probe, the fixing portion and the measured object. The first posture positioning feature may be arranged on at least one selected from the measurement probe, the fixing seat, the measured object, or other objects. An adjustment process implemented based on the optical method will be described below from two aspects, including an arrangement position of the structure for projecting the second projection feature and an arrangement position of the first posture positioning feature.

A description will be given below from the aspect of the arrangement position of the structure for projecting the second projection feature.

First, if the structure for projecting the second projection feature is arranged on the measured object, the first posture positioning feature may be arranged on at least one selected from the measured object, the measurement probe, the fixing seat, or other objects. It should be noted that if the first posture positioning feature is arranged on the measurement probe, in order to achieve the positioning of the measurement posture, it is required that the position of the measurement probe is fixed in the stage of the first positioning of the measurement posture. Similarly, if the first posture positioning feature is arranged on the fixing seat, in order to achieve the positioning of the measurement posture, it is required that the position of the fixing seat is fixed in the stage of the first positioning of the measurement posture.

Second, if the structure for projecting the second projection feature is arranged on the measurement probe, the first posture positioning feature may not be arranged on the measurement probe, but may be arranged on the measured object, the fixing seat or other objects. It should be noted that the position of the measurement probe needs to be fixed in the stage of the first positioning of the measurement posture. In addition, if the first posture positioning feature is arranged on the fixing seat, the first positioning of the measurement posture may be achieved by the method of adjusting the current measurement posture of the measured object according to the first posture positioning feature and the second projection feature until the first posture positioning feature is matched with the second projection feature. The first posture positioning feature being matched with the second projection feature here means that the first posture positioning feature is blocked by the measured object, so that the second projection feature may not be projected to the position where the first posture positioning feature is located. If the first posture positioning feature is not matched with the second posture positioning feature, at least one second projection feature may be projected to the position where the first posture positioning feature is located. If the first posture positioning feature is arranged on other objects, the positioning of the measurement posture may be achieved by a method similar to that of arranging the first posture positioning feature on the fixing seat, and details will not be repeated here.

Third, if the structure for projecting the second projection feature is arranged on the fixing seat, the first posture positioning feature may not be arranged on the fixing seat, but may be arranged on the measured object, the measurement probe or other objects. It should be noted that the position of the fixing seat may be fixed in the stage of the first positioning of the measurement posture. In addition, if the first posture positioning feature is arranged on the measurement probe or other objects, the positioning of the measurement posture may be achieved by a method similar to that of arranging the structure for projecting the second projection feature on the measurement probe and arranging the first posture positioning feature on the fixing seat or other objects, and details will not be repeated here.

Fourth, if the structure for projecting the second projection feature is arranged on other objects, the first posture positioning feature may be arranged on at least one selected from the measured object, the measurement probe, the fixing seat, or other objects. It should be noted that if the first posture positioning feature is arranged on the measurement probe, the fixing seat or other objects, the positioning of the measurement posture may be achieved by a method similar to that of arranging the first posture positioning feature on the fixing seat or other objects, and details will not be repeated here..

A description will be given below from the aspect of the arrangement position of the first posture positioning feature.

First, if the first posture positioning feature is arranged on the measured object, the structure for projecting the second projection feature may be arranged on the measured object, the measurement probe, the fixing seat or other objects. It should be noted that if the structure for projecting the second projection feature is arranged on the measurement probe, the position of the measurement probe needs to be fixed in the stage of the first positioning of the measurement posture. Similarly, if the structure for projecting the second projection feature is arranged on the fixing seat, the position of the fixing seat needs to be fixed in the stage of the first positioning of the measurement posture.

Second, if the first posture positioning feature is arranged on the measurement probe, the structure for projecting the second projection feature is separated from the measurement probe, and may be arranged on the measured object, the fixing seat or other objects. It should be noted that if the structure for projecting the second projection feature is arranged on the measured object, the fixing seat or other objects, reference may be made to the above description of the corresponding part, and details will not be repeated here.

Third, if the first posture positioning feature is arranged on the fixing seat, the structure for projecting the second projection feature is separated from the fixing seat, and may be arranged on the measured object, the measurement probe or other objects. It should be noted that if the structure for projecting the second projection feature is arranged on the measured object, the measurement probe or other objects, reference may be made to the above description of the corresponding part, and details will not be repeated here.

Fourth, if the first posture positioning feature is arranged on other objects, the structure for projecting the second projection feature may be arranged on the measured object, the measurement probe, the fixing seat or other objects. It should be noted that if the structure for projecting the second projection feature is arranged on the measured object, the measurement probe, the fixing seat or other objects, reference may be made to the above description of the corresponding part, and details will not be repeated here.

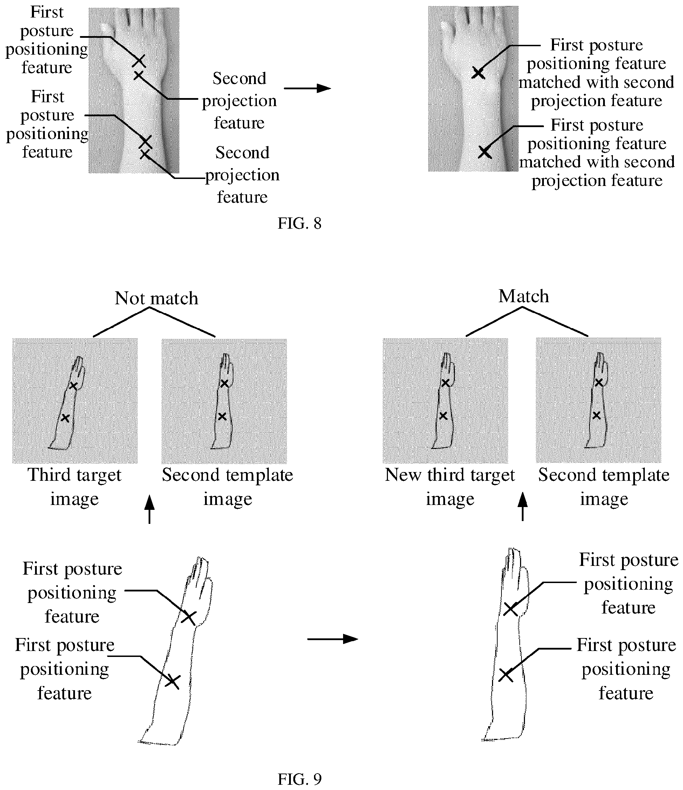

Exemplarily, FIG. 8 schematically shows a schematic diagram of positioning the measurement posture based on an optical method according to embodiments of the present disclosure. In FIG. 8, the first posture positioning feature is arranged on the measured object.

When positioning the measurement posture using an optical method, on the one hand, a position and an angle of the light source may be adjusted flexibly and may be easily matched with the first posture positioning feature. Therefore, the first posture positioning feature may be arranged flexibly, so that the difficulty in the arrangement of the first posture positioning feature is reduced. On the other hand, the shape of the exit light spot may be adjusted to be matched with the first posture positioning feature better, so that the positioning accuracy may be improved.

According to embodiments of the present disclosure, adjusting the current measurement posture of the measured object to the target measurement posture according to the first posture positioning feature may include the following operations.

A third target image is obtained. A second template image is obtained, where the second template image contains the first posture positioning feature. When it is determined that the third target image is not matched with the second template image, the current measurement posture is adjusted to obtain a new third target image until the new third target image is matched with the second template image. When it is determined that the new third target image is matched with the second template image, it is determined that the current measurement posture is the target measurement posture.

According to embodiments of the present disclosure, in order to ensure the flexibility of use and the positioning accuracy of the measurement posture, it is possible to adopt an image matching method, that is, to match the third target image with the second template image, and determine the target measurement posture according to a matching result. The second template image may contain the first posture positioning feature, and the position of the first posture positioning feature in the second template image is a predetermined position. In the process of matching the third target image with the second template image, the third target image may be a target image that does not contain the first posture positioning feature, or may be a target image that contains the first posture positioning feature but the position of the first posture positioning feature in the third target image is not the predetermined position, or may be a target image that contains the first posture positioning feature and the position of the first posture positioning feature in the third target image is the predetermined position. Since the second template image contains the first posture positioning feature at the predetermined position, if the third target image is matched with the second template image, it may indicate that the third target image contains the first posture positioning feature and the position of the first posture positioning feature in the third target image is the predetermined position. In other words, a purpose of matching the third target image with the second template image is to obtain such third target image that the first posture positioning feature is contained in the third target image and the position of the first posture positioning feature in the third target image is the predetermined position.

According to embodiments of the present disclosure, a structure for acquiring the third target image may be arranged on the measured object, the measurement probe, the fixing seat or other objects. Other objects may refer to objects other than the measurement probe, the fixing portion and the measured object. The first posture positioning feature may be arranged on at least one selected from the measurement probe, the fixing seat, the measured object, or other objects. For the description of the first posture positioning feature and the structure for acquiring the third target image, reference may be made to the description of the first posture positioning feature and the structure for projecting the second projection feature, and details will not be repeated here. A difference is that the first posture positioning feature may be arranged on at least one selected from the measured object, the measurement probe, the fixing seat, or other objects if the structure for acquiring the third target image is arranged on the measurement probe; the first posture positioning feature may be arranged on at least one selected from the measured object, the measurement probe, the fixing seat, or other objects if the structure for acquiring the third target image is arranged on the fixing seat.

Exemplarily, FIG. 9 schematically shows a schematic diagram of positioning the measurement posture based on an image matching method according to embodiments of the present disclosure. In FIG. 9, the first posture positioning feature is arranged on the measured object.

According to embodiments of the present disclosure, adjusting the current measurement posture of the measured object to the target measurement posture according to the first posture positioning feature may include the following operations.

A fourth target image is obtained, where the fourth target image contains the first posture positioning feature. When it is determined that the position of the first posture positioning feature in the fourth target image is not a second predetermined position, the current measurement posture is adjusted to obtain a new fourth target image until the position of the first posture positioning feature in the new fourth target image is the second predetermined position. When it is determined that the position of the first posture positioning feature in the new fourth target image is the second predetermined position, it is determined that the current measurement posture is the target measurement posture.

According to embodiments of the present disclosure, in order to ensure the flexibility of use and the positioning accuracy of the measurement posture, it is possible to adopt an imaging method, that is, if the position of the first posture positioning feature in the fourth target image is the second predetermined position, it may indicate that the positioning of the measurement posture is completed.

According to embodiments of the present disclosure, the process of positioning the measurement posture using the imaging method is a process of determining whether the position of the first posture positioning feature in the fourth target image is the second predetermined position. If the position of the first posture positioning feature in the four target image is not the second predetermined position, the current measurement posture may be adjusted to obtain a new fourth target image until the position of the first posture positioning feature in the new fourth target image is the second predetermined position. When the position of the first posture positioning feature in the new fourth target image is the second predetermined position, it may indicate that the current measurement posture is the target measurement posture.

According to embodiments of the present disclosure, a structure for acquiring the fourth target image may be arranged on the measured object, the measurement probe, the fixing seat or other objects. Other objects may refer to objects other than the measurement probe, the fixing portion and the measured object. The first posture positioning feature may be arranged on at least one selected from the measurement probe, the fixing seat, the measured object, or other objects. For the description of the first posture positioning feature and the structure for acquiring the fourth target image, reference may be made to the description of the first posture positioning feature and the structure for projecting the second projection feature, and details will not be repeated here.

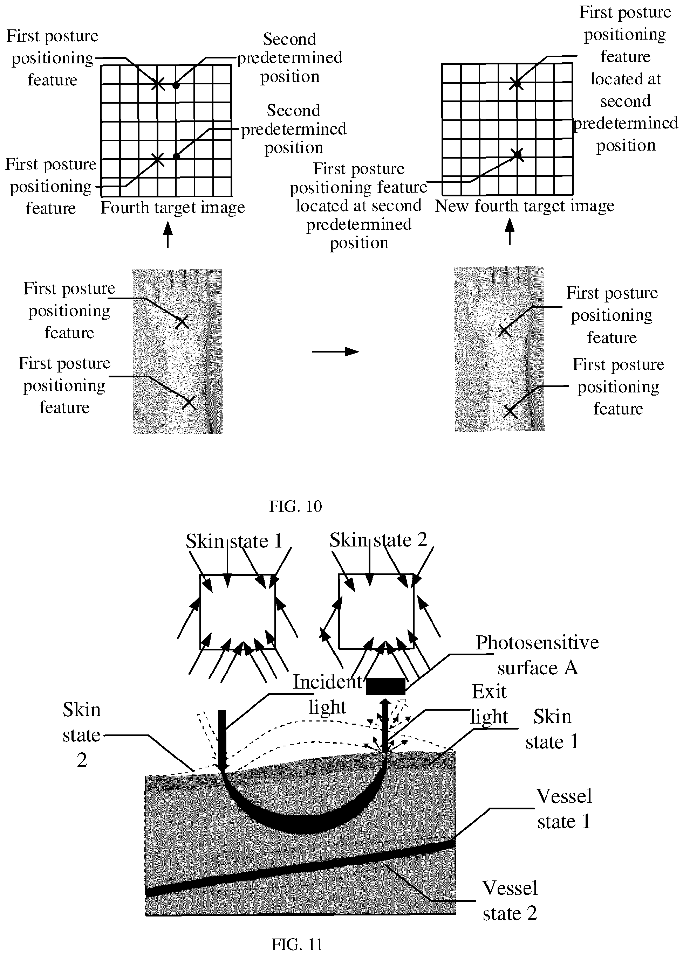

Exemplarily, FIG. 10 schematically shows a schematic diagram of positioning the measurement posture based on an imaging method according to embodiments of the present disclosure. In FIG. 10, the first posture positioning feature is arranged on the measured object.

According to embodiments of the present disclosure, the method may further include the following operations.

If the measurement probe is arranged at the position corresponding to the measurement region, the second posture positioning feature is determined when it is determined that the current measurement posture is not the target measurement posture. The current measurement posture is adjusted to the target measurement posture according to the second posture positioning feature.

According to embodiments of the present disclosure, if the measurement probe is arranged at the position corresponding to the measurement region, the above-mentioned second positioning of the measurement posture needs to be performed when it is determined that the current measurement posture is not the target measurement posture. That is, after the positioning of the measurement region is completed, the above-mentioned second positioning of the measurement posture needs to be performed if the current measurement posture is not the target measurement posture. The current measurement posture may be adjusted according to the second posture positioning feature until the current measurement posture is the target measurement posture. The second posture positioning feature may be the same as or different from the first posture positioning feature.

According to embodiments of the present disclosure, adjusting the current measurement posture to the target measurement posture according to the second posture positioning feature may include the following operations.

A third projection feature is obtained. When it is determined that the second posture positioning feature is not matched with the third projection feature, the current measurement posture is adjusted until the second posture positioning feature is matched with the third projection feature. When it is determined that the second posture positioning feature is matched with the third projection feature, it is determined that the current measurement posture is the target measurement posture.

According to embodiments of the present disclosure, in order to ensure the flexibility of use and the positioning accuracy of the measurement posture, it is possible to adopt an optical method, that is, to match the second posture positioning feature with the third projection feature, and determine the target measurement posture according to a matching result. The third projection feature is formed according to an optical method, that is, by projecting a light spot with a predetermined shape using a light source. The shape of the light spot may be determined according to the second posture positioning feature. That is, for the measured object, the third projection feature matched with the second posture positioning feature is arranged according to the second posture positioning feature, and the current measurement posture with which the second posture positioning feature is matched with the third projection feature is the target measurement posture.

According to embodiments of the present disclosure, a structure for projecting the third projection feature may be arranged on the measured object, the measurement probe, the fixing seat or other objects. Other objects may refer to objects other than the measurement probe, the fixing portion and the measured object. The second posture positioning feature may be arranged on at least one selected from the measurement probe, the fixing seat, the measured object, or other objects. An adjustment process implemented based on the optical method will be described below from two aspects, including an arrangement position of the structure for projecting the third projection feature and an arrangement position of the second posture positioning feature.

A description will be given below from the aspect of the arrangement position of the structure for projecting the third projection feature.

First, if the structure for projecting the third projection feature is arranged on the measured object, the second posture positioning feature may be arranged on at least one selected from the measured object, the measurement probe, the fixing seat, or other objects.

Second, if the structure for projecting the third projection feature is arranged on the measurement probe, the second posture positioning feature may not be arranged on the measurement probe and the fixing seat, but may be arranged on the measured object or other objects, because the measurement probe is located on the fixing seat after it is arranged at the position corresponding to the measurement region.

Third, if the structure for projecting the third projection feature is arranged on the fixing seat, the second posture positioning feature may not be arranged on the measurement probe and the fixing seat, but may be arranged on the measured object or other objects, because the measurement probe is located on the fixing seat after it is arranged at the position corresponding to the measurement region.

Fourth, if the structure for projecting the third projection feature is arranged on other objects, the second posture positioning feature may be arranged on at least one selected from the measured object, the measurement probe, the fixing seat, or other objects. It should be noted that if the second posture positioning feature is arranged on other objects, the positioning of the measurement posture may be achieved by the method of adjusting the current measurement posture when it is determined that the second posture positioning feature is not matched with the third projection feature, until the second posture positioning feature is matched with the third projection feature, and determining that the current measurement posture is the target measurement posture when it is determined that the second posture positioning feature is matched with the third projection feature. The second posture positioning feature being matched with the third projection feature here means that the second posture positioning feature is blocked by the measured object, so that the third projection feature may not be projected to the position where the second posture positioning feature is located. If the second posture positioning feature is not matched with the third projection feature, at least one third projection feature may be projected to the position where the second posture positioning feature is located.

A description will be given below from the aspect of the arrangement position of the second posture positioning feature.

First, if the second posture positioning feature is arranged on the measured object, the structure for projecting the third projection feature may be arranged on the measured object, the measurement probe, the fixing seat or other objects.

Second, if the second posture positioning feature is arranged on the measurement probe, the structure for projecting the third projection feature is separated from the measurement probe and the fixing seat, and may be arranged on the measured object or other objects, because the measurement probe is located on the fixing seat after it is arranged at the position corresponding to the measurement region.

Third, if the second posture positioning feature is arranged on the fixing seat, then the structure for projecting the third projection feature is separated from the measurement probe and the fixing seat, and may be arranged on the measured object or other objects, because the measurement probe is located on the fixing seat after it is arranged at the position corresponding to the measurement region.

Fourth, if the second posture positioning feature is arranged on other objects, the structure for projecting the third projection feature may be arranged on the measured object, the measurement probe, the fixing seat or other objects. It should be noted that if the structure for projecting the third projection feature is arranged on other objects, reference may be made to the above description of the corresponding part, and details will not be repeated here.

When positioning the measurement posture using an optical method, on the one hand, a position and an angle of the light source may be adjusted flexibly and may be easily matched with the second posture positioning feature. Therefore, the second posture positioning feature may be arranged flexibly, so that the difficulty in the arrangement of the second posture positioning feature is reduced. On the other hand, the shape of the exit light spot may be adjusted to be matched with the second posture positioning feature better, so that the positioning accuracy may be improved.

According to embodiments of the present disclosure, adjusting the current measurement posture to the target measurement posture according to the second posture positioning feature may include the following operations.

A fifth target image is obtained. A third template image is obtained, where the third template image contains the second posture positioning feature. When it is determined that the fifth target image is not matched with the third template image, the current measurement posture is adjusted to obtain a new fifth target image until the new fifth target image is matched with the third template image. When it is determined that the new fifth target image is matched with the third template image, it is determined that the current measurement posture is the target measurement posture.

According to embodiments of the present disclosure, in order to ensure the flexibility of use and the positioning accuracy of the measurement posture, it is possible to adopt an image matching method, that is, to match the fifth target image with the third template image, and determine the target measurement posture according to a matching result. The third template image may contain the second posture positioning feature, and the position of the second posture positioning feature in the third template image is a predetermined position. In the process of matching the fifth target image with the third template image, the fifth target image may be a target image that does not contain the second posture positioning feature, or may be a target image that contains the second posture positioning feature but the position of the second posture positioning feature in the fifth target image is not the predetermined position, or may be a target image that contains the second posture positioning feature and the position of the second posture positioning feature in the fifth target image is the predetermined position. Since the third template image contains the second posture positioning feature at the predetermined position, if the fifth target image is matched with the third template image, it may indicate that the fifth target image contains the second posture positioning feature and the position of the second posture positioning feature in the fifth target image is the predetermined position. In other words, a purpose of matching the fifth target image with the third template image is to obtain such fifth target image that the second posture positioning feature is contained in the fifth target image and the position of the second posture positioning feature in the fifth target image is the predetermined position.

According to embodiments of the present disclosure, when it is determined that the fifth target image is matched with the third template image, it may indicate that the current measurement posture is the target measurement posture.

According to embodiments of the present disclosure, a structure for acquiring the fifth target image may be arranged on the measured object, the measurement probe, the fixing seat, or other objects. Other objects may refer to objects other than the measurement probe, the fixing portion and the measured object. The second posture positioning feature may be arranged on at least one selected from the measurement probe, the fixing seat, the measured object, or other objects. For the description of the second posture positioning feature and the structure for acquiring the fifth target image, reference may be made to the description of the second posture positioning feature and the structure for projecting the third projection feature, and details will not be repeated here. A difference is that the second posture positioning feature may be arranged on at least one selected from the measured object, the measurement probe, the fixing seat, or other objects if the structure for acquiring the fifth target image is arranged on the measurement probe; the second posture positioning feature may be arranged on at least one selected from the measured object, the measurement probe, the fixing seat, or other objects if the structure for acquiring the fifth target image is arranged on the fixing seat.

According to embodiments of the present disclosure, adjusting the current measurement posture to the target measurement posture according to the second posture positioning feature may include the following operations.

A sixth target image is obtained, where the sixth target image contains the second posture positioning feature. When it is determined that the position of the second posture positioning feature in the sixth target image is not a third predetermined position, the current measurement posture is adjusted to obtain a new sixth target image until the position of the second posture positioning feature in the new sixth target image is the third predetermined position. When it is determined that the position of the second posture positioning feature in the new sixth target image is the third predetermined position, it is determined that the current measurement posture is the target measurement posture.

According to embodiments of the present disclosure, in order to ensure the flexibility of use and the positioning accuracy of the measurement posture, it is possible to adopt an imaging method, that is, if the position of the second posture positioning feature in the sixth target image is the third predetermined position, it may indicate that the positioning of the measurement posture is completed.

According to embodiments of the present disclosure, the process of positioning the measurement posture using the imaging method is a process of determining whether the position of the second posture positioning feature in the sixth target image is the third predetermined position. If the position of the second posture positioning feature in the sixth target image is not the third predetermined position, the current measurement posture may be adjusted to obtain a new sixth target image until the position of the second posture positioning feature in the new sixth target image is the third predetermined position. When the position of the second posture positioning feature in the new sixth target image is the third predetermined position, it may indicate that the current measurement posture is the target measurement posture.

According to embodiments of the present disclosure, a structure for acquiring the sixth target image may be arranged on the measured object, the measurement probe, the fixing seat, or other objects. Other objects may refer to objects other than the measurement probe, the fixing portion and the measured object. The second posture positioning feature may be arranged on at least one selected from the measurement probe, the fixing seat, the measured object, or other objects. For the description of the second posture positioning feature and the structure for acquiring the sixth target image, reference may be made to the description of the second posture positioning feature and the structure for projecting the third projection feature, and details will not be repeated here.

According to embodiments of the present disclosure, the method may further include the following operations.

A prompt information is generated. The prompt information is used to prompt a completion of the positioning of the measurement posture and/or a completion of the positioning of the measurement region. A form of the prompt information includes at least one selected from an image, a speech, or a vibration.

According to embodiments of the present disclosure, in order to allow a user to timely know whether the positioning of the measurement posture and/or the positioning of the measurement region are/is completed, the prompt information may be generated after the completion of the positioning of the measurement posture and/or the completion of the positioning of the measurement region. A specific representation form of the prompt information may include at least one selected from an image, a speech, or a vibration.

According to embodiments of the present disclosure, the method may further include the following operations.

The measurement probe is arranged on the fixing seat when it is determined that the fixing seat is arranged at the position corresponding to the measurement region and that the measurement probe is not arranged on the fixing portion. When it is determined that the fixing seat is not arranged at the position corresponding to the measurement region, the fixing seat is arranged at the position corresponding to the measurement region by the first fitting part, and the measurement probe is arranged on the fixing seat.

According to embodiments of the present disclosure, if the measurement probe is arranged at the position corresponding to the measurement region by the fixing seat, then during the tissue element measurement process, the fixing seat may be detached from the measurement region, and the measurement probe may be detached from the fixing seat. When it is required to perform a measurement, if the fixing seat is not arranged at the position corresponding to the measurement region, then the fixing seat may be arranged at the position corresponding to the measurement region by the first fitting part, and the measurement probe may be arranged on the fixing seat. If the fixing seat is arranged at the position corresponding to the measurement region and the measurement probe is not arranged on the fixing seat, then the measurement probe may be arranged on the fixing seat.

Exemplarily, for a short-term measurement at any time, the fixing seat may be arranged at the position corresponding to the measurement region, and the measurement probe may be detached from the fixing seat; when it is required to perform a measurement, the measurement probe may be arranged on the fixing seat. For a long-term measurement, the fixing seat may be detached from the measurement region, and the measurement probe may be detached from the fixing seat; when it is required to perform a measurement, the fixing seat may be arranged at the position corresponding to the measurement region by the first fitting part, and the measurement probe may be arranged on the fixing seat.

According to embodiments of the present disclosure, the method may further include the following operations.

When it is determined that the measurement probe is not arranged at the position corresponding to the measurement region, the measurement probe is arranged at the position corresponding to the measurement region by the second fitting part.

According to embodiments of the present disclosure, if the measurement probe is directly arranged at the position corresponding to the measurement region, then during the tissue element measurement process, the measurement probe may be detached from the measurement region, and when it is required to perform a measurement, the measurement probe may be arranged at the position corresponding to the measurement region by the second fitting part.

According to embodiments of the present disclosure, performing the tissue element measurement using the measurement probe may include the following operations.

The measurement region is irradiated by incident light having at least one predetermined wavelength, where each beam of the incident light is incident on an incident position to form at least one beam of exit light exited from at least one exit position on the measurement region. A light intensity value corresponding to each beam of the exit light acquired by the measurement probe is obtained, so as to obtain T output light intensities, where the measurement probe includes M photosensitive surfaces, and each output light intensity is obtained by processing the light intensity value of the exit light acquired by one or more photosensitive surfaces, and 1≤T≤M. The concentration of the measured tissue element is determined according to at least one output light intensity corresponding to the at least one predetermined wavelength.

According to embodiments of the present disclosure, as different measurement sites have different skin characteristics, including smoothness, hair presence or absence, flatness, skin thickness and softness, etc., it is needed to select an appropriate measurement site according to an actual situation, such as a structure of the measurement probe. The measurement site may include at least one selected from a finger, a palm, an arm, a forehead, or an earlobe. The measurement region may be a region on the measurement site.

According to embodiments of the present disclosure, the predetermined wavelength may be a wavelength sensitive to the measured tissue element. A band to which the predetermined wavelength belongs may include an ultraviolet band, a visible light band, a near-infrared band, a mid-infrared band or a far-infrared band. Exemplarily, if the measured tissue element is blood glucose, the single predetermined wavelength may be a wavelength sensitive to blood glucose accordingly, which may be specifically 1550 nm or 1609 nm. The incident light may be collimated or non-collimated. There may be one or more incident positions of the incident light.

According to embodiments of the present disclosure, each of the M photosensitive surfaces may be used separately, partially in combination, or all in combination. Used in combination means outputting one output light intensity. In embodiments of the present disclosure, a photosensitive surface for outputting one output light intensity is referred to as a homogeneous photosensitive surface. The homogeneous photosensitive surface may include one or more photosensitive surfaces. A condition for using different photosensitive surfaces in combination may be that for each photosensitive surface, an average optical path of the exit light received by the photosensitive surface is within an average optical path range. The average optical path range may be a range that is greater than or equal to a first average optical path threshold and less than or equal to a second average optical path threshold. The first average optical path threshold and the second average optical path threshold may be determined according to an optical path mean value and an optical path change amplitude. The optical path mean value is a mean value calculated according to the average optical paths of the exit light received at the photosensitive positions of the homogeneous photosensitive surface. Exemplarily, if the optical path mean value is a, and the optical path change amplitude is ±30%, then the first average optical path threshold may be 0.7a, and the second average optical path threshold may be 1.3a.

The average optical path is explained as follows. A transmission path of light in a tissue may be expressed by an optical path and a penetration depth. The optical path is used to represent a total distance that light travels in the tissue, and the penetration depth is used to represent a maximum longitudinal distance that light may reach in the tissue. For a determined source-detection distance, the average optical path is used to represent an average value of the optical path of light in the tissue. A probability distribution function of the optical path may be understood as a function of the source-detection distance and a tissue optical parameter. The source-detection distance represents a radial distance between a center of the incident light and a center of the photosensitive surface. Accordingly, in terms of mathematical expressions, the average optical path may be understood as a function of the source-detection distance and the tissue optical parameter. The tissue optical parameter may include an absorption coefficient, a scattering coefficient, and an anisotropy factor. A factor affecting the average optical path may include the absorption coefficient, the scattering coefficient, the anisotropy factor, and the source-detection distance.

According to embodiments of the present disclosure, the homogeneous photosensitive surface may be a ring photosensitive surface or a non-ring photosensitive surface. The homogeneous photosensitive surface being a ring photosensitive surface may include that the homogeneous photosensitive surface includes one photosensitive surface, and the homogeneous photosensitive surface is an independent ring photosensitive surface; or the homogeneous photosensitive surface includes a plurality of photosensitive surfaces, and the homogeneous photosensitive surface is a ring photosensitive surface formed by combining the plurality of photosensitive surfaces. The homogeneous photosensitive surface being a non-ring photosensitive surface may include that the homogeneous photosensitive surface includes one photosensitive surface, and the homogeneous photosensitive surface is an independent non-ring photosensitive surface; or the homogeneous photosensitive surface includes a plurality of photosensitive surfaces, and the homogeneous photosensitive surface is a non-ring photosensitive surface formed by combining the plurality of photosensitive surfaces.

After at least one output light intensity corresponding to the predetermined wavelength is obtained, the at least one output light intensity may be processed using a differential measurement method to determine the concentration of the measured tissue element. The differential measurement method may include a temporal differential measurement method, a position differential measurement method, or a wavelength differential measurement method. Alternatively, the at least one output light intensity may also be processed using a non-differential measurement method to determine the concentration of the measured tissue element.

According to embodiments of the present disclosure, determining the concentration of the measured tissue element according to the at least one output light intensity corresponding to the at least one predetermined wavelength may include the following operations.

For each predetermined wavelength in the at least one predetermined wavelength, a first output light intensity and a second output light intensity are determined from two output light intensities corresponding to the predetermined wavelength. A differential processing is performed on the first output light intensity and the second output light intensity corresponding to the predetermined wavelength to obtain a differential signal. The concentration of the measured tissue element is determined according to the differential signal corresponding to each predetermined wavelength.

According to embodiments of the present disclosure, the average optical path of the exit light corresponding to the first output light intensity is different from the average optical path of the exit light corresponding to the second output light intensity. The differential processing performed on the first output light intensity and the second output light intensity corresponding to the predetermined wavelength may include a processing method in terms of hardware and a processing method in terms of software. The processing method in terms of hardware may include processing by using a differential circuit. The processing method in terms of software may include performing a differential operation by using a differential algorithm. The differential algorithm may include a direct differential operation and a logarithmic differential operation. The direct differential operation refers to directly performing a differential processing on two parameters. The logarithmic differential operation refers to performing a logarithmic operation on two parameters to obtain logarithmic parameters, and then performing a differential processing on the two logarithmic parameters.

According to embodiments of the present disclosure, each photosensitive surface may acquire the light intensity value of the exit light exited from the exit position within the predetermined anti-jitter range corresponding to the photosensitive surface.

According to embodiments of the present disclosure, in the process of implementing concepts of the present disclosure, the inventors further found that: different measurement results may be obtained if only an intensity distribution of a light spot irradiated by the incident light on the measurement region is changed while other conditions remain unchanged; and if a measurement result obtained when arranging a photosensitive surface close to a blood vessel is compared with a measurement result obtained when arranging the same photosensitive surface far away from the blood vessel while keeping other conditions unchanged, the measurement result obtained when arranging the photosensitive surface far away from the blood vessel is better than that obtained when arranging the photosensitive surface close to the blood vessel. The measurement result may be represented by a relative variation of a light intensity value of exit light received by the photosensitive surface or a standard deviation of the light intensity value. The less the relative variation of the light intensity value, the better the measurement result, and the less the standard deviation of the light intensity value, the better the measurement result. When studying the causes for the different measurement results, it was found that changing the intensity distribution of the light spot irradiated by the incident light on the measurement region may reflect a randomness of an irradiation of a light source, and a distance between the measurement region and the blood vessel may reflect a strength of a pulse beat. The randomness of the irradiation of the light source and the pulse beat are both sources of jitter. Therefore, it has been found that one of the causes for the low measurement accuracy is the jitter.

On the basis of a research on the jitter, it was found that a source of the jitter may be classified into an internal source and an external source. The internal source may further include a change in the physiological background in addition to the pulse beat. The external source may further include an uncertainty of a transmission of the incident light in addition to the randomness of the irradiation of the light source. The randomness of the irradiation of the light source may be reflected by the intensity distribution of the light spot irradiated by the incident light on the measurement region. It was found that both the jitter caused by the internal source and the jitter caused by the external source may affect a transmission path of light in the tissue, and then affect the intensity distribution of the exit light on the measurement region.

In order to solve the problem of the difficulty in obtaining a true signal of the measured tissue element caused by the jitter, the inventors found that a solution of acquiring the light intensity value of the exit light by using a large-area photosensitive surface may be adopted to effectively reduce an adverse influence of the jitter on the measurement result. That is, the large-area photosensitive surface may effectively reduce the adverse influence caused by the jitter. The so-called "large-area photosensitive surface" may be understood as a photosensitive surface with such area that the photosensitive surface may acquire the light intensity value of the exit light exited from the exit position within the predetermined anti-jitter range. A description will be given below to explain in detail why the solution of acquiring the output light intensity of the exit light by using the large-area photosensitive surface may be adopted to effectively reduce the adverse influence of the jitter on the measurement result.

In the large-area photosensitive surface, a ratio of an area of the photosensitive surface that may stably receive the exit light to the area of the photosensitive surface may be increased, so that a stability of receiving the exit light may be improved, an adverse influence of a change in the intensity distribution of the exit light caused by the jitter may be reduced, and then the possibility of obtaining the true signal of the measured tissue element may be improved. The stability may be represented by a relative variation of the light intensity value of the exit light received by the photosensitive surface or a standard deviation of the light intensity value. The less the relative variation of the light intensity value, the higher the stability, and the less the standard deviation of the light intensity value, the higher the stability.

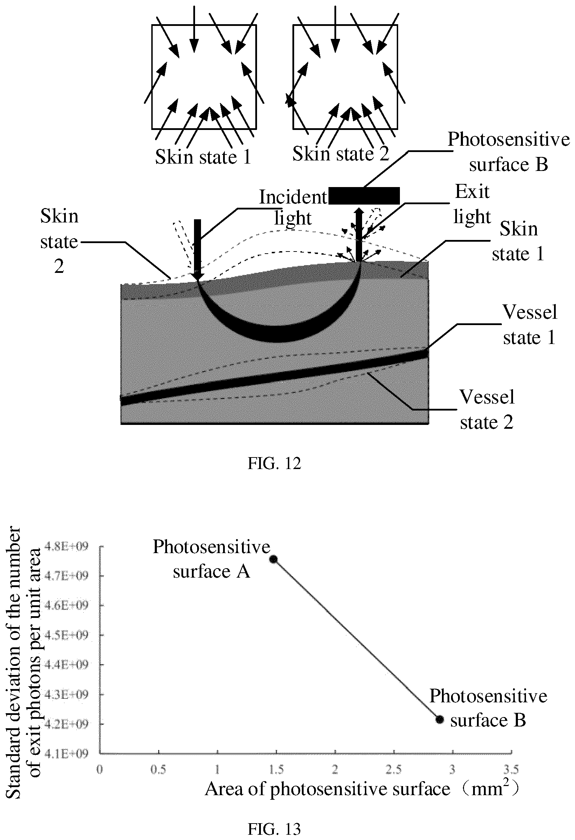

Illustratively, the jitter caused by the pulse beat is taken as an example for description. The pulse beat may be reflected by a blood vessel state. FIG. 11 schematically shows a schematic diagram of receiving the exit light using a photosensitive surface with a small area when a jitter occurs according to embodiments of the present disclosure. FIG. 12 schematically shows a schematic diagram of receiving the exit light using a photosensitive surface with a large area when a jitter occurs according to embodiments of the present disclosure. The jitter occurs in FIG. 11 is the same as that occurs in FIG. 12. An area of a photosensitive surface A in FIG. 11 is smaller than an area of a photosensitive surface B in FIG. 12, and both the photosensitive surface A and the photosensitive surface B are square photosensitive surfaces. In FIG. 11 and FIG. 12, a blood vessel state 1 represents a vasoconstriction state, a blood vessel state 2 represents a vasodilation state, a skin state 1 represents a skin state corresponding to the blood vessel state 1, and a skin state 2 represents a skin state corresponding to the blood vessel state 2. A change from the skin state 1 to the skin state 2 reflects a jitter.

The measurement results obtained using photosensitive surfaces with different areas when the same jitter occurs are compared. The measurement result is represented by the relative variation of the light intensity value of the exit light received by the photosensitive surface within a predetermined time period or the standard deviation of the light intensity value. The relative variation of the light intensity value may be determined by: calculating a difference between a maximum light intensity value and a minimum light intensity value within the predetermined time period, calculating an average value of the light intensity values within the predetermined time period, calculating a ratio of the difference to the average value, and determining the ratio as the relative variation of the light intensity value. The predetermined time period may be a pulsation cycle.

The measurement results also show that the measurement result obtained by using the photosensitive surface B is better than the measurement result obtained by using the photosensitive surface A whether the measurement result is represented by the relative variation of the light intensity value of the exit light received by the photosensitive surface or the measurement result is represented by the standard deviation of the light intensity value of the exit light received by the photosensitive surface.

As the area of the photosensitive surface B is larger than the area of the photosensitive surface A, it may indicate that the large-area photosensitive surface may improve the stability of receiving the exit light, and then reduce the adverse influence of the change in the intensity distribution of the exit light caused by the jitter, thereby improving the possibility of obtaining the true signal of the measured tissue element.

It should be noted that the large-area photosensitive surface described in embodiments of the present disclosure may achieve a high stability and efficiency of receiving the exit light in a case of a small distance to a surface of the measurement region, that is, in a case that the large-area photosensitive surface is close to the surface of the measurement region. This may not be achieved by using a single-point optical fiber reception and a joint reception of multiple single optical fibers due to a constraint of a numerical aperture of the optical fiber and a limitation of a state change of the optical fiber. The state of the optical fiber is easily affected by an environment, and the state change of the optical fiber has a great influence on the stability of receiving the exit light.

It should also be noted that the large-area photosensitive surface is generally adopted to improve the signal-to-noise ratio of the output light intensity. In other words, a large-area photosensitive surface may not only improve the efficiency of the output light intensity, but also effectively suppress the jitter.

In order to improve the reliability of the measurement result, it is needed to ensure that each photosensitive surface may acquire the light intensity value of the exit light exited from the exit position within the predetermined anti-jitter range corresponding to the photosensitive surface, which requires the area of the photosensitive surface to be as large as possible. Each photosensitive surface has a corresponding predetermined anti-jitter range, and different photosensitive surfaces correspond to the same or different predetermined anti-jitter ranges. A description will be given below from three aspects with examples to illustrate that the larger the area of the photosensitive surface, the better the effect of suppressing the jitter. In the examples, the area of the photosensitive surface A is smaller than the area of the photosensitive surface B, and both the photosensitive surface A and the photosensitive surface B are square photosensitive surfaces.

In a first aspect, a jitter caused by a pulse beat may be suppressed. The photosensitive surface A and the photosensitive surface B are respectively arranged at a same position on the measurement region, which is a position close to the blood vessel. A measurement result obtained by using the photosensitive surface A may be compared with a measurement result obtained by using the photosensitive surface B when other conditions are the same. The measurement result is represented by a relative variation of the light intensity value of the exit light received by the photosensitive surface within a pulsation cycle or a standard deviation of the light intensity value. The method of calculating the relative variation of the light intensity value is as described above, and will not be repeated here. It was found that the relative variation of the light intensity value of the exit light received by the photosensitive surface B is less than the relative variation of the light intensity value of the exit light received by the photosensitive surface A, and the standard deviation of the light intensity value of the exit light received by the photosensitive surface B is less than the standard deviation of the light intensity value of the exit light received by the photosensitive surface A. Therefore, it may be concluded that the measurement result obtained by using the photosensitive surface B is better than the measurement result obtained by using the photosensitive surface A whether the measurement result is represented by the relative variation of the light intensity value of the exit light received by the photosensitive surface or the measurement result is represented by the standard deviation of the light intensity value of the exit light received by the photosensitive surface.

Since the measurement result obtained by using the photosensitive surface B is better than the measurement result obtained by using the photosensitive surface A, and the area of the photosensitive surface B is larger than the area of the photosensitive surface A, it may indicate that the larger the area of the photosensitive surface, the better the effect of suppressing the jitter caused by the pulse beat.

In a second aspect, a jitter caused by a change in the intensity distribution of the light spot irradiated by the incident light on the measurement region may be suppressed. The intensity distribution of the light spot irradiated by the incident light on the measurement region may be changed while other conditions remain unchanged. The measurement result obtained by using the photosensitive surface A may be compared with the measurement result obtained by using the photosensitive surface B. The measurement result is represented by a relative variation of the light intensity value of the exit light received by the photosensitive surface within a predetermined period or a standard deviation of the light intensity value. The method of calculating the relative variation of the light intensity value is as described above, and will not be repeated here. It was found that the relative variation of the light intensity value of the exit light received by the photosensitive surface B is less than the relative variation of the light intensity value of the exit light received by the photosensitive surface A, and the standard deviation of the light intensity value of the exit light received by the photosensitive surface B is less than the standard deviation of the light intensity value of the exit light received by the photosensitive surface A. Therefore, it may be concluded that the measurement result obtained by using the photosensitive surface B is better than the measurement result obtained by using the photosensitive surface A whether the measurement result is represented by the relative variation of the light intensity value of the exit light received by the photosensitive surface or the measurement result is represented by the standard deviation of the light intensity value of the exit light received by the photosensitive surface.

Since the measurement result obtained by using the photosensitive surface B is better than the measurement result obtained by using the photosensitive surface A, and the area of the photosensitive surface B is larger than the area of the photosensitive surface A, it may indicate that the larger the area of the photosensitive surface, the better the effect of suppressing the jitter caused by the change in the intensity distribution of the light spot irradiated by the incident light on the measurement region.

In a third aspect, a jitter caused by an uncertainty of the transmission of the incident light may be suppressed. A Monte Carlo simulation method is used, where a central incidence is performed using incident light with a photon number of 10¹⁵, the photosensitive surface A and the photosensitive surface B are respectively arranged at a distance of 2.4 mm from the center of the incident light, and the simulation is performed twenty-two times. The measurement result obtained by using the photosensitive surface A may be compared with the measurement result obtained by using the photosensitive surface B. The measurement result is represented by a standard deviation of a number of exit photons per unit area. The less the standard deviation of the number of exit photons per unit area, the better the suppression effect. FIG. 13 schematically shows a schematic diagram of the measurement result obtained based on the Monte Carlo simulation method according to embodiments of the present disclosure. It was found that the standard deviation of the number of exit photons per unit area corresponding to the photosensitive surface B is less than the standard deviation of the number of exit photons per unit area corresponding to the photosensitive surface A. That is, the measurement result obtained by using the photosensitive surface B is better than the measurement result obtained by using the photosensitive surface A.

Since the measurement result obtained by using the photosensitive surface B is better than the measurement result obtained by using the photosensitive surface A, and the area of the photosensitive surface B is larger than the area of the photosensitive surface A, it may indicate that the larger the area of the photosensitive surface, the better the effect of suppressing the jitter caused by the uncertainty of the transmission of the incident light.

Through the examples from the above three aspects, it may indicate that the larger the area of the photosensitive surface, the better the effect of suppressing the adverse influence of the jitter on the measurement result.

According to embodiments of the present disclosure, a ratio of an average optical path of the exit light received by each photosensitive surface in a target tissue layer to a total optical path is greater than or equal to a ratio threshold. The total optical path is a total distance that the exit light travels in the measurement region.

According to embodiments of the present disclosure, a tissue model of the measured object is generally a layered structure, that is, it may be divided into one or more layers. Different tissue layers carry different information of the measured tissue element. In order to improve the possibility of obtaining the true signal of the measured tissue element, it is required to ensure that the transmission path of the exit light mainly passes through the tissue layer that carries a rich information of the measured tissue element. A target tissue layer may be understood as the tissue layer that carries a rich information of the measured tissue element, or the tissue layer that is a main source of the measured tissue element. In the following description, a human body is taken as an example of the measured object, and blood glucose is taken as an example of the measured tissue element.

A skin tissue model of the human body may be understood as a three-layer model, including epidermis, dermis and subcutaneous fat layer from outside to inside. The epidermis contains a small amount of interstitial fluid, and does not contain plasma and lymph. The dermis contains a large amount of interstitial fluid, and further contains a large amount of plasma and a small amount of lymph due to the presence of abundant capillaries. The subcutaneous fat layer contains a small amount of cellular fluid, and contains a large amount of plasma and a small amount of lymph due to the presence of blood vessels such as veins and arteries. Therefore, different tissue layers carry different information of the measured tissue element.

Since the epidermis contains a small amount of interstitial fluid, it is not an appropriate source of the blood glucose information. Although the subcutaneous fat layer contains a large amount of plasma and a relatively small amount of interstitial fluid, it is still not an appropriate source of the blood glucose information due to a limitation of the penetration depth of the incident light. The dermis contains abundant capillaries and a large amount of interstitial fluid, and the incident light may easily reach the dermis. Therefore, the dermis may be used as a main source of the blood glucose information. Accordingly, the target tissue layer may be the dermis.

According to embodiments of the present disclosure, the average optical path of the exit light in each tissue layer may be determined according to the optical path and the penetration depth.

In order to ensure that the transmission path of the exit light mainly passes through the target tissue layer, it is required to ensure that a ratio of the average optical path of the exit light received by each photosensitive surface in the target tissue layer to the total optical path is greater than or equal to a ratio threshold. The total optical path may be a total distance that the exit light travels in the measurement region, that is, a total distance of a path that the incident light travels from entering the measurement region, travelling in the measurement region, to reaching the exit position. The ratio threshold is related to the tissue optical parameter and the source-detection distance between the center of the photosensitive surface and the center of the incident light.

It should be noted that, since the ratio of the average optical path of the exit light received by the photosensitive surface in the target tissue layer to the total optical path is limited in embodiments of the present disclosure, the area of the photosensitive surface in embodiments of the present disclosure may not be overly large, and it is a large area in an area range.

According to embodiments of the present disclosure, the method may further include the following operations.

A total area of the homogeneous photosensitive surface is determined according to a tissue structure feature in the measurement region. The homogeneous photosensitive surface includes one or more photosensitive surfaces, and the homogeneous photosensitive surface is used to output one output light intensity.

According to embodiments of the present disclosure, the total area of the homogeneous photosensitive surface may be determined according to the tissue structure feature in the measurement region. The tissue structure feature may be understood as a structure feature of the measurement region.

For example, when the measurement region is a region where three blood vessels intersect, if the homogeneous photosensitive surface is arranged in the region where the three blood vessels intersect, the total area of the homogeneous photosensitive surface is limited to an area of the region where the three blood vessels intersect, that is, the total area of the homogeneous photosensitive surface needs to be determined according to the area of the region where the three blood vessels intersect.

For another example, when the measurement region is a region where a finger is located, if the homogeneous photosensitive surface is arranged in the region where the finger is located, the total area of the homogeneous photosensitive surface is limited by an area of the region where the finger is located, that is, the total area of the homogeneous photosensitive surface needs to be determined according to the area of the region where the finger is located.

It should be noted that the area of the photosensitive surface in embodiments of the present disclosure may be determined according to the tissue structure feature, and an area that is determined according to the tissue structure feature may generally not be overly large. Therefore, the area of the photosensitive surface in embodiments of the present disclosure may not be overly large, and it is a large area in the area range.

According to embodiments of the present disclosure, a ratio of the area of each photosensitive surface to a circumference of the photosensitive surface is greater than or equal to a ratio threshold.

According to embodiments of the present disclosure, the reason why the influence of the jitter caused by the uncertainty of the transmission of the incident light, the randomness of the light source, the change in the physiological background and the pulse beat on the distribution of the exit light on the measurement region may be reduced by maximizing the ratio of the area of the photosensitive surface to the circumference of the photosensitive surface is as follows.

For ease of explanation, the photosensitive surface is divided into two portions, including an edge portion and a non-edge portion (or inner portion). Generally, the jitter mainly affects the exit light acquired by the edge portion, while the non-edge portion is less affected, that is, the non-edge portion may acquire the exit light relatively stably. From another perspective, when the jitter occurs, the intensity distribution of the exit light on the measurement region may change slightly. The light intensity value of the exit light received by the edge portion may change greatly with the change in the intensity distribution of the exit light, but most of the exit light at the non-edge portion may be acquired by the photosensitive surface relatively stably, so that the light intensity value of the exit light received by the non-edge portion may remain relatively stable. Therefore, it is possible to maximize a ratio of an area corresponding to the non-edge portion to the area of the photosensitive surface in order to effectively suppress the adverse influence of the jitter on the measurement result, and the larger the ratio, the better the effect of reducing the adverse influence. The edge portion may be represented by the circumference of the photosensitive surface, and the non-edge portion may be represented by the area of the photosensitive surface. Thus, the ratio of the area of the photosensitive surface to the circumference of the photosensitive surface is required to be as large as possible.

For example, a photosensitive surface 1 is a circular photosensitive surface, and a photosensitive surface 2 is a square photosensitive surface. In a case of a same circumference, since the area of the photosensitive surface 1 is larger than the area of the photosensitive surface 2, the ratio of the area of the photosensitive surface 1 to the circumference of the photosensitive surface 1 is greater than the ratio of the area of the photosensitive surface 2 to the circumference of the photosensitive surface 2. Therefore, the effect of the photosensitive surface 1 reducing the adverse influence is better than the effect of the photosensitive surface 2 reducing the adverse influence.

It should be noted that the description for the ratio of the area of the photosensitive surface to the circumference of the photosensitive surface being greater than or equal to the ratio threshold is given on the basis of meeting the condition that the area of the photosensitive surface is greater than or equal to the area threshold. For most shapes of photosensitive surfaces, if the ratio of the area of the photosensitive surface to the circumference of the photosensitive surface is greater than or equal to the ratio threshold, a size of the area of the photosensitive surface is actually limited, because for most shapes of figures, the ratio of the area to the circumference of the figure has a positive correlation with the size of the area, that is, the greater the ratio of the area to the circumference of the figure, the larger the area of the figure. For example, in a case of a circle, an area of the circle is *πR*², and a ratio of the area to the circumference of the circle is R12, where R represents a radius. The ratio of the area to the circumference of the circle is only related to the radius, and the size of the area of the circle is only related to the radius. Therefore, the ratio of the area to the circumference of the circle has a positive correlation with the size of the area. If the ratio of the area to the circumference of the circle is limited, the size of the area of the circle is also limited. For another example, in a case of a square, an area of the square is *a*², and a ratio of the area to the circumference of the square is *a*/4, where a represents a side length. The ratio of the area to the circumference of the square is only related to the side length, and the size of the area of the square is only related to the side length. Therefore, the ratio of the area to the circumference of the square has a positive correlation with the size of the area. If the ratio of the area to the circumference of the square is limited, the size of the area of the square is also limited.

According to embodiments of the present disclosure, the ratio threshold is greater than or equal to 0.04 mm.

According to embodiments of the present disclosure, the area of the photosensitive surface in the present disclosure is a relatively large area, that is, the area of the photosensitive surface is a large area within an area range. A description will be given below for this case.

First, the area of the photosensitive surface may not be too small. The large-area photosensitive surface in embodiments of the present disclosure refers to a photosensitive surface with such area that the photosensitive surface may acquire the light intensity value of the exit light exited from the exit position within the predetermined anti-jitter range. Therefore, the large-area photosensitive surface in embodiments of the present disclosure has a large area used to achieve anti-jitter. Furthermore, the ratio of the area of the photosensitive surface to the circumference of the photosensitive surface may be used to indicate that the area of the photosensitive surface allows the photosensitive surface to acquire the light intensity value of the exit light exited from the exit position within the predetermined anti-jitter range, and the ratio of the area to the circumference of the photosensitive surface generally has a positive correlation with the area of the photosensitive surface. Therefore, if the ratio of the area to the circumference of the photosensitive surface is greater than or equal to the ratio threshold, the size of the area of the photosensitive surface is also actually limited, that is, it is also limited that the area of the photosensitive surface may not be too small by limiting that the ratio of the area to the circumference of the photosensitive surface is greater than or equal to the ratio threshold.

Second, the area of the photosensitive surface may not be overly large. In embodiments of the present disclosure, it is required that the ratio of the average optical path of the exit light received by the photosensitive surface in the target tissue layer to the total optical path is greater than or equal to the ratio threshold, and/or the area of the photosensitive surface is determined according to the tissue structure feature, so that the area of the photosensitive surface may not be overly large.

Therefore, the area of the photosensitive surface in embodiments of the present disclosure is a relatively large area, that is, a large area within the area range.

In addition, there may be a case that the photosensitive surface has a large area and also has a large circumference, so that the ratio of the area of the photosensitive surface to the circumference of the photosensitive surface is small, that is, the ratio of the area of the photosensitive surface to the circumference of the photosensitive surface is less than the ratio threshold. Therefore, it is possible that a photosensitive surface having a large absolute area does not meet the anti-jitter requirement. There may also be a case that the photosensitive surface has a small area and a large circumference, so that the ratio of the area of the photosensitive surface to the circumference of the photosensitive surface is less than the ratio threshold. Therefore, a photosensitive area having a very small area may not meet the anti-jitter requirement.

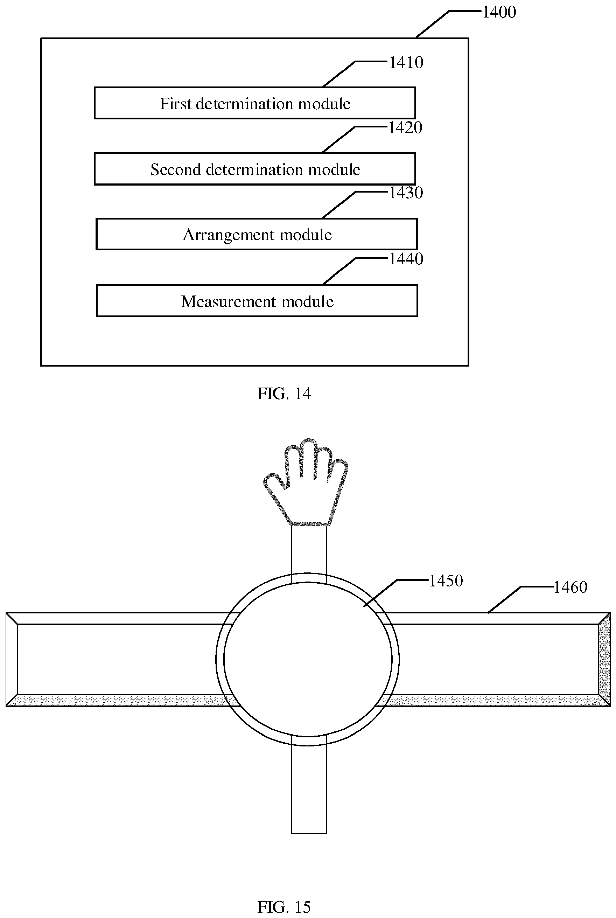

FIG. 14 schematically shows a block diagram of a device of measuring a tissue element according to embodiments of the present disclosure.

As shown in FIG. 14, a device 1400 of measuring a tissue element further includes a first determination module 1410, a second determination module 1420, an arrangement module 1430, and a measurement module 1440.

The first determination module 1410 is used to determine a positioning feature.

The second determination module 1420 is used to determine a measurement region according to the positioning feature, where the measurement region is a region that meets a reproducibility of a measurement condition.

The arrangement module 1430 is used to arrange a measurement probe 1450 at a position corresponding to the measurement region.

The measurement module 1440 is used to perform a tissue element measurement by using the measurement probe 1450.

According to the technical solutions of embodiments of the present disclosure, by determining the positioning feature, determining the measurement region meeting the reproducibility of the measurement condition according to the positioning feature, arranging the measurement probe at the position corresponding to the measurement region, and performing a tissue element measurement by using the measurement probe, the measurement region may be positioned accurately, and then the reproducibility of the measurement condition may be effectively controlled.

According to embodiments of the present disclosure, the positioning feature includes a first posture positioning feature and a region positioning feature.

The second determination module 1420 includes a first adjustment unit and a first determination unit.

The first adjustment unit is used to adjust a current measurement posture of the measured object to a target measurement posture according to the first posture positioning feature. The target measurement posture is a measurement posture that meets the reproducibility of the measurement condition. The first determination unit is used to determine the measurement region according to the region positioning feature when the current measurement posture is the target measurement posture.

As shown in FIG. 15, according to embodiments of the present disclosure, the device 1400 of measuring the tissue element further includes a fixing portion 1460 used to arrange the measurement probe 1450 at the position corresponding to the measurement region. The fixing portion 1460 is integrated with, partially separated from or completely separated from the measurement probe 1450.

According to embodiments of the present disclosure, the fixing portion 1460 and the measurement probe 1450 in FIG. 15 may be integrated or separated.

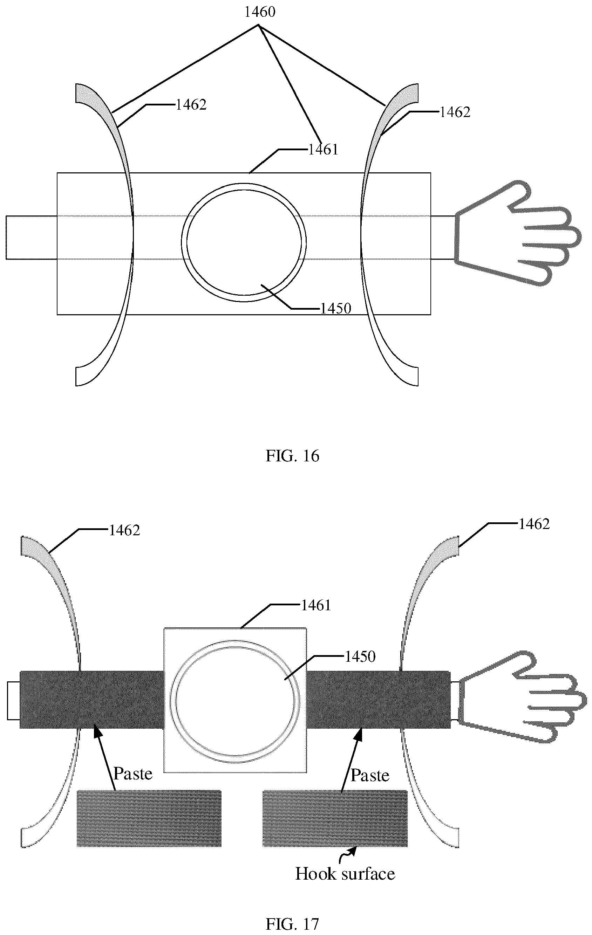

As shown in FIG. 16, according to embodiments of the present disclosure, the fixing portion 1460 includes a fixing seat 1461 and a first fitting part 1462.

The first fitting part 1462 is used to arrange the fixing seat 1461 at the position corresponding to the measurement region. The fixing seat 1461 is used to fix the measurement probe 1450.

According to embodiments of the present disclosure, a hardness of the first fitting part 1462 includes a first hardness and a second hardness. The first hardness is less than the second hardness. The first hardness is a corresponding hardness in a process of fixing the fixing seat 1461 by the first fitting part 1462, and the second hardness is a corresponding hardness after the fixing seat 1461 is fixed by the first fitting part 1462.

According to embodiments of the present disclosure, in order to enable the first fitting part 1462 to fix the fixing seat 1461, the first fitting part 1462 is required to be rigid. Furthermore, in order to minimize an influence produced when fixing the fixing seat 1461 by the first fitting part 1462, the first fitting part 1462 is required to have a certain flexibility. Therefore, a requirement is put forward on the hardness of the first fitting part 1462.

In order to solve the above problems, it is possible to adopt a method of changing the hardness of the first fitting part 1462, that is, the hardness of the first fitting part 1462 includes the first hardness and the second hardness. The first hardness represents the corresponding hardness in the process of fixing the fixing seat 1461 by the first fitting part 1462, the second hardness represents the corresponding hardness after the fixing seat 1461 is fixed by the first fitting part 1462, and the first hardness is less than the second hardness. In this way, it may not only ensure that the first fitting part achieves the fixation function, but also minimize the influence produced when fixing the fixing seat 1461 by the first fitting part 1462.

According to embodiments of the present disclosure, the first fitting part 1462 includes a first Velcro or a first elastic belt.

Exemplarily, FIG. 17 schematically shows a schematic diagram of the first fitting part according to embodiments of the present disclosure. The first fitting part 1462 in FIG. 17 is a Velcro. A material of a loop surface of the Velcro is very soft, so that the influence produced when fixing the fixing seat 1461 by the first fitting part 1462 may be reduced. At this time, the hardness of the first fitting part 1462 is the first hardness. Meanwhile, in order to enable the first fitting part 1462 to achieve the fixation function, after the fixing seat 1461 is fixed by the first fitting part 1462, a hook surface may be pasted on the loop surface to increase the hardness of the first fitting part 1462. At this time, the hardness of the first fitting part 1462 is the second hardness.

According to embodiments of the present disclosure, the corresponding hardness in the process of fixing the fixing seat 1461 by the first fitting part 1462 is the first hardness, which may reduce the influence produced when fixing the fixing seat 1461 by the first fitting part 1462. Therefore, it may be ensured as much as possible that the skin state of the skin at the measurement region meets a first predetermined condition in the process of arranging the fixing seat 1461 at the position corresponding to the measurement region by the first fitting part 1462.

According to embodiments of the present disclosure, a surface of the first fitting part 1462 is provided with a hole.

According to embodiments of the present disclosure, the hardness of the first fitting part 1462 is greater than or equal to a first hardness threshold and less than or equal to a second hardness threshold.

According to embodiments of the present disclosure, in order to meet the hardness requirement of the first fitting part 1462, in addition to the method described above, it is also possible to adopt a method of manufacturing the first fitting part 1462 using a material having a hardness greater than or equal to the first hardness threshold and less than or equal to the second hardness threshold, which may also enable the first fitting part 1462 to fix the fixing seat 1461 while minimizing the influence produced when fixing the fixing seat 1461 by the first fitting part 1462. It should be noted that the first hardness threshold and the second hardness threshold may be determined according to actual situations, and are not specifically limited here.

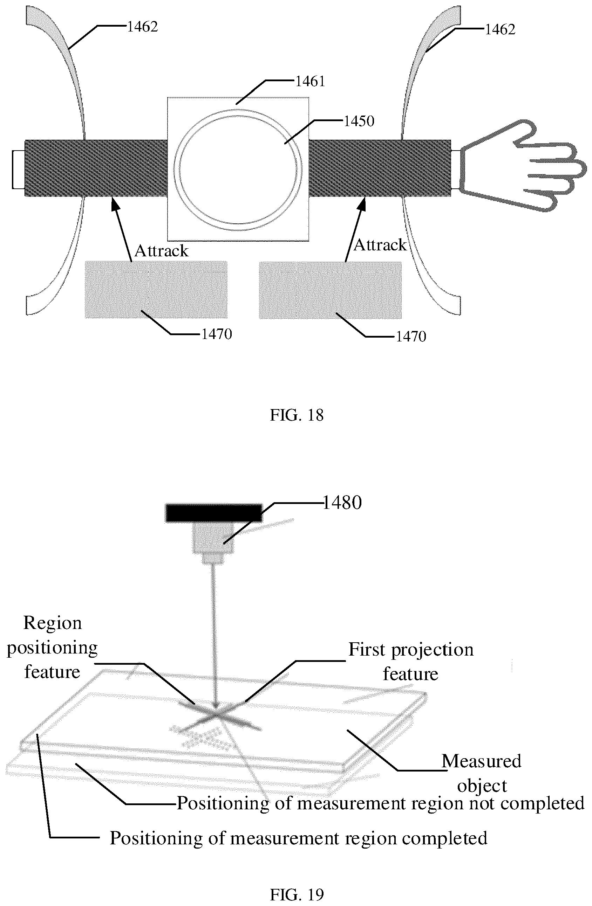

As shown in FIG. 18, according to embodiments of the present disclosure, the device 1400 of measuring the tissue element may further include a first magnetic portion 1470. The first fitting part 1462 is entirely or partially a metal hinge, and the first magnetic portion 1470 fits with the first fitting part 1462 to fix the fixing seat 1461.

According to embodiments of the present disclosure, in order to meet the hardness requirement of the first fitting part 1462, in addition to the method described above, it is also possible to adopt a method of designing the first fitting part 1462 to be entirely or partially a metal hinge, which may also enable the first fitting part 1462 to fix the fixing seat 1461 while minimizing the influence produced when fixing the fixing seat 1461 by the first fitting part 1462.

The fixation function may be achieved as follows. After the first fitting part 1462 completes the fixing of the fixing seat 1461, the first magnetic portion 1470 may be attracted to the first fitting part 1462, so that the first magnetic portion 1470 fits with the first fitting part 1462 to fix the fixing seat 1461, thereby achieving the fixation function. Referring to FIG. 18, FIG. 18 schematically shows a schematic diagram of another first fitting part 1462 according to embodiments of the present disclosure. The first fitting part 1462 in FIG. 18 is entirely a metal hinge. The first magnetic portion 1470 may be attracted to the first fitting part 1462 after the first fitting part 1462 completes the fixing of the fixing seat 1461. The magnetic portion 1470 may be a miniature electromagnetic portion 1470.

In addition, since the metal hinge is a ferromagnetic metal and a metal is easy to absorb heat, a direct contact between the metal hinge and the skin may produce a great influence on a skin temperature. In order to avoid an influence of a heat absorption of the metal on the skin temperature, it is possible to place a thermal insulator under the metal hinge. Optionally, the thermal insulator may be flannelette.

The above may be achieved because a good flexibility of the metal hinge may reduce the influence produced when fixing the fixing seat 1461 by the first fitting part 1462. Moreover, after the first fitting part 1462 completes the fixing of the fixing seat 1461, since the first magnetic portion is attracted on the first fitting part 1462, a cooperation of the two makes the first fitting part 1462 become harder, so that the fixation function may be achieved.

It should be noted that the first fitting part 1462 is entirely or partially a metal hinge, and a good flexibility of the metal hinge may reduce the influence produced when fixing the fixing seat 1461 by the first fitting part 1462. Therefore, it may be ensured as much as possible that the skin state of the skin at the measurement region meets the first predetermined condition in the process of arranging the fixing seat 1461 at the position corresponding to the measurement region by the first fitting part 1462.

According to embodiments of the present disclosure, the measurement probe 1450 is fixed on the fixing seat 1461 in at least one manner selected from: the measurement probe 1450 being fixed on the fixing seat 1461 by an adhesive tape; The measurement probe 1450 being fixed on the fixing seat 1461 by a fastener; the measurement probe 1450 being fixed on the fixing seat 1461 by a magnetic force; or a friction coefficient between the measurement probe 1450 and the fixing seat 1461 being greater than or equal to a friction coefficient threshold.

According to embodiments of the present disclosure, in order to fix the measurement probe 1450 on the fixing seat 1461 and ensure that the measurement probe 1450 does not move on the fixing seat 1461, at least one of the following methods may be adopted.

In a first method, the measurement probe 1450 may be fixed on the fixing seat 1461 by an adhesive tape. In a second method, the measurement probe 1450 may be fixed on the fixing seat 1461 by a fastener. In a third method, the measurement probe 1450 may be fixed on the fixing seat 1461 by a magnetic force. In a fourth method, the friction coefficient between the measurement probe 1450 and the fixing seat 1461 may be greater than or equal to the friction coefficient threshold. Optionally, a material of the fixing seat 1461 is rubber.

According to embodiments of the present disclosure, the fixing portion 1460 includes a second fitting part.

The second fitting part is used to arrange the measurement probe 1450 at the position corresponding to the measurement region.

According to embodiments of the present disclosure, a hardness of the second fitting part includes a third hardness and a fourth hardness. The third hardness is less than the fourth hardness. The third hardness is a corresponding hardness in a process of fixing the measurement probe 1450 by the second fitting part, and the fourth hardness is a corresponding hardness after the measurement probe 1450 is fixed by the second fitting part.

According to embodiments of the present disclosure, the second fitting part includes a second Velcro or a second elastic belt.

According to embodiments of the present disclosure, a surface of the second fitting part is provided with a hole.

According to embodiments of the present disclosure, the hardness of the second fitting part is greater than or equal to a third hardness threshold and less than or equal to a fourth hardness threshold.

According to embodiments of the present disclosure, for the relevant description of the second fitting part, reference may be made to the above description of the first fitting part 1462, and details will not be repeated here. A difference is that the second fitting part is used to fix the measurement probe 1450.

According to embodiments of the present disclosure, the first determination unit is used to obtain a first projection feature. When it is determined that the region positioning feature is not matched with the first projection feature, the position of the measurement probe 1450 and/or the fixing portion 1460 is adjusted until the region positioning feature is matched with the first projection feature. When it is determined that the region positioning feature is matched with the first projection feature, a region corresponding to the measurement probe 1450 and/or the fixing portion 1460 is determined as the measurement region.

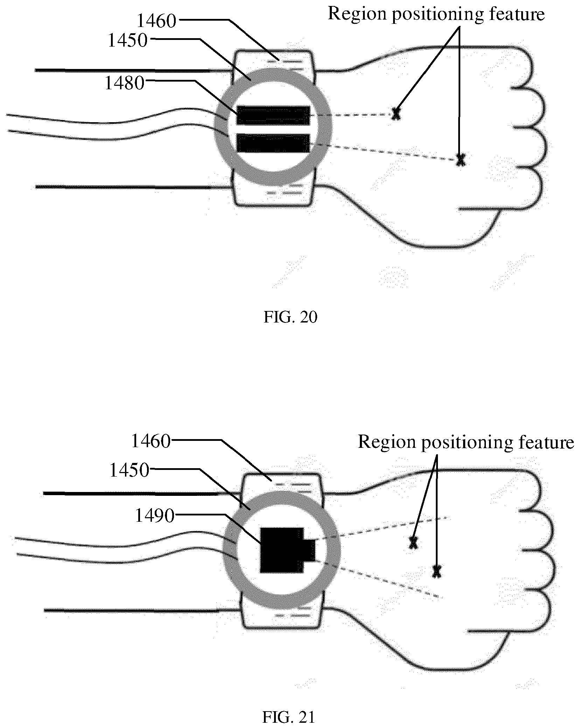

As shown in FIG. 19 and FIG. 20, according to embodiments of the present disclosure, the device 1400 of measuring the tissue element further includes a region positioning portion 1480 arranged on the measured object, the measurement probe 1450, the fixing portion 1460 or other objects. The region positioning portion 1480 is used to project the first projection feature.

According to embodiments of the present disclosure, when it is determined that the region positioning portion 1480 is arranged on the measurement probe 1450, the region positioning feature is not arranged on the measurement probe 1450. When it is determined that the region positioning portion 1480 is arranged on the fixing portion 1460, the region positioning feature is not arranged on the fixing portion 1460.

According to embodiments of the present disclosure, FIG. 19 schematically shows a schematic diagram of a region positioning portion 1480 according to embodiments of the present disclosure. The measurement probe 1450 and the fixing portion 1460 are not shown in FIG. 19. The region positioning portion 1480 is used to project the first projection feature, which is a cross light spot. The region positioning feature is a cross marker.

FIG. 20 schematically shows a schematic diagram of another region positioning portion 1480 according to embodiments of the present disclosure. In FIG. 20, the region positioning portion 1480 is integrated with the measurement probe 1450 and the fixing portion 1460, and the region positioning feature is arranged on a back of a hand of the measured object. The region positioning portion 1480 is used to project the first projection feature, and the first projection feature is a cross light spot.

According to embodiments of the present disclosure, the region positioning portion 1480 includes a first laser.

According to embodiments of the present disclosure, the first laser may project a light spot having a predetermined shape, so as to form the first projection feature.

According to embodiments of the present disclosure, the first determination unit is used to obtain a first target image, and obtain a first template image, where the first template image contains the region positioning feature. When it is determined that the first target image is not matched with the first template image, the position of the measurement probe 1450 and/or the fixing portion 1460 is adjusted to obtain a new first target image until the new first target image is matched with the first template image. When it is determined that the first target image is matched with the first template image, the region corresponding to the measurement probe 1450 and/or the fixing portion 1460 is determined as the measurement region.

As shown in FIG. 21, according to embodiments of the present disclosure, the device 1400 of measuring the tissue element further includes a first image acquisition portion 1490 arranged on the measured object, the measurement probe 1450, the fixing portion 1460 or other objects. The first image acquisition portion 1490 is used to acquire the first target image.

According to embodiments of the present disclosure, FIG. 21 schematically shows a schematic diagram of a first image acquisition portion according to embodiments of the present disclosure. In FIG. 21, the first image acquisition portion 1490 is integrated with the measurement probe 1450 and the fixing portion 1460, and the region positioning feature is arranged on the back of the hand of the measured object. The first image acquisition portion 1490 is used to acquire the first target image. The first image acquisition portion 1490 may be an image sensor.

According to embodiments of the present disclosure, the first determination unit is used to obtain a second target image, where the second target image contains the region positioning feature. When it is determined that the position of the region positioning feature in the second target image is not the first predetermined position, the position of the measurement probe 1450 and/or the fixing portion 1460 is adjusted to obtain a new second target image until the position of the region positioning feature in the second target image is the first predetermined position. When it is determined that the position of the region positioning feature in the new second target image is the first predetermined position, the region corresponding to the measurement probe 1450 and/or the fixing portion 1460 is determined as the measurement region.

According to embodiments of the present disclosure, the device 1400 of measuring the tissue element further includes a second image acquisition portion arranged on the measured object, the measurement probe 1450, the fixing unit 1460 or other objects. The second image acquisition portion is used to acquire the second target image.

According to embodiments of the present disclosure, when it is determined that the second image acquisition portion is arranged on the measurement probe 1450, the region positioning feature is not arranged on the measurement probe 1450. When it is determined that the second image acquisition portion is arranged on the fixing portion 1460, the region positioning feature is not arranged on the fixing portion 1460.

According to embodiments of the present disclosure, the second image acquisition portion is the same as or different from the first image acquisition portion 1490.

According to embodiments of the present disclosure, the first adjustment unit is used to obtain a second projection feature. When it is determined that the first posture positioning feature is not matched with the second projection feature, the current measurement posture is adjusted until the first posture positioning feature is matched with the second projection feature. When it is determined that the first posture positioning feature is matched with the second projection feature, it is determined that the current measurement posture is the target measurement posture.

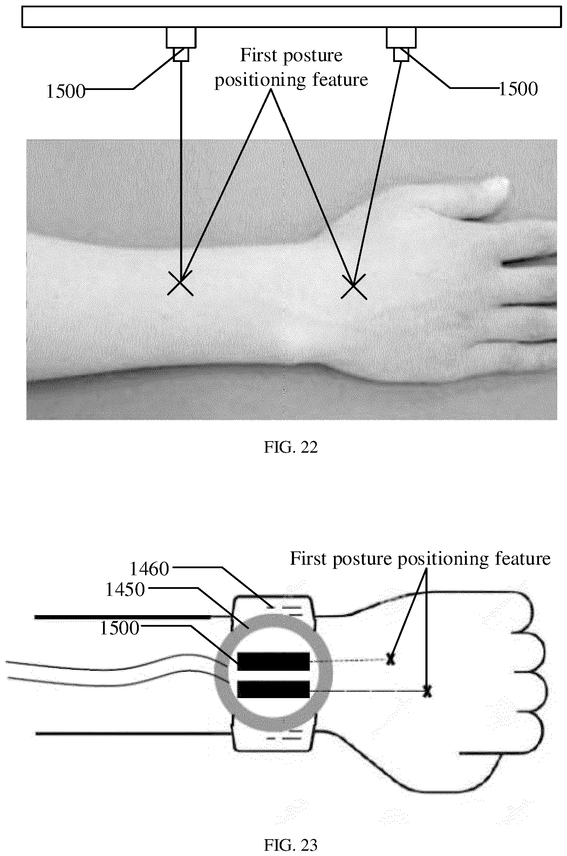

As shown in FIG. 22 to FIG. 23, according to embodiments of the present disclosure, the device 1400 of measuring the tissue element measurement further includes a first posture positioning portion 1500 arranged on the measured object, the measurement probe 1450, the fixing portion 1460 or other objects. The first posture positioning portion 1500 is used to project the second projection feature.

According to embodiments of the present disclosure, when it is determined that the first posture positioning portion 1500 is arranged on the measurement probe 1450, the first posture positioning feature is not arranged on the measurement probe 1450. When it is determined that the first posture positioning portion 1500 is arranged on the fixing portion 1460 , the first posture positioning feature is not arranged on the fixing portion 1460.

According to embodiments of the present disclosure, FIG. 22 schematically shows a schematic diagram of a first posture positioning portion according to embodiments of the present disclosure. The measurement probe 1450 and the fixing portion 1460 are not shown in FIG. 22. The first posture positioning portion 1500 is used to project the second projection feature, which is a cross light spot. The first posture positioning feature is a cross marker.

FIG. 23 schematically shows a schematic diagram of another first posture positioning portion according to embodiments of the present disclosure. In FIG. 23, the first posture positioning portion 1500 is integrated with the measurement probe 1450 and the fixing portion 1460, and the first posture positioning feature is arranged on the back of the hand of the measured object. The first posture positioning portion 1500 is used to project the second projection feature, which is a cross light spot.

According to embodiments of the present disclosure, the first posture positioning portion 1500 includes a second laser.

According to embodiments of the present disclosure, the second laser may project a light spot having a predetermined shape, so as to form the second projection feature.

According to embodiments of the present disclosure, the first adjustment unit is used to obtain a third target image, and obtain a second template image, where the second template image contains the first posture positioning feature. When it is determined that the third target image is not matched with the second template image, the current measurement posture is adjusted to obtain a new third target image until the new third target image is matched with the second template image. When it is determined that the new third target image is matched with the second template image, it is determined that the current measurement posture is the target measurement posture.

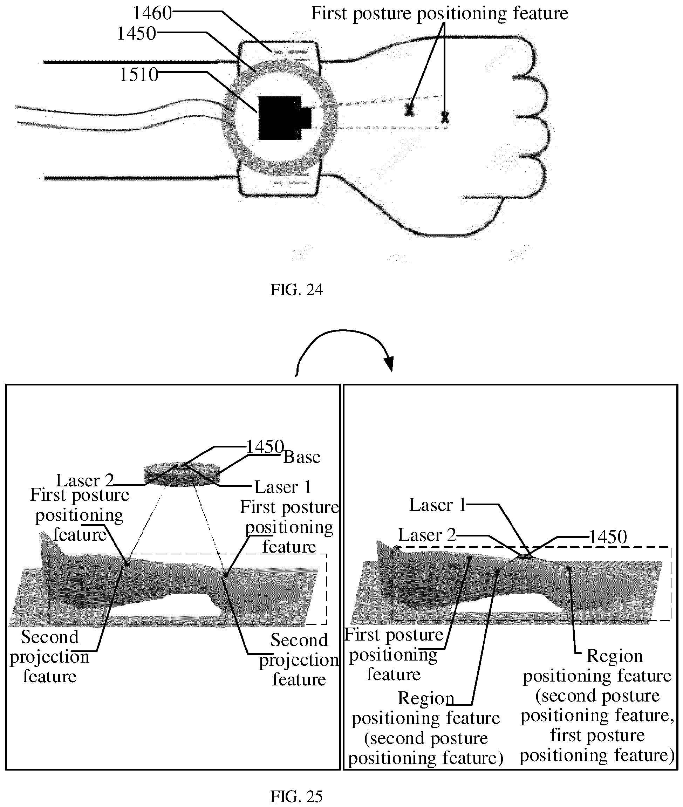

As shown in FIG. 24, according to embodiments of the present disclosure, the device 1400 of measuring the tissue element further includes a third image acquisition portion 1510 arranged on the measured object, the measurement probe 1450, the fixing portion 1460 or other objects. The third image acquisition portion 1510 is used to acquire the third target image.

According to embodiments of the present disclosure, FIG. 24 schematically shows a schematic diagram of the third image acquisition portion according to embodiments of the present disclosure. In FIG. 24, the third image acquisition portion 1510 is integrated with the measurement probe 1450 and the fixing portion 1460, and the first posture positioning feature is arranged on the back of the hand of the measured object. The third image acquisition portion 1510 is used to acquire the third target image. The third image acquisition portion 1510 may be an image sensor.

According to embodiments of the present disclosure, the third image acquisition portion 1510, the first image acquisition portion 1490 and the second image acquisition portion may be different, partially the same, or completely the same.

According to embodiments of the present disclosure, the first adjustment unit is used to obtain a fourth target image, where the fourth target image contains the first posture positioning feature. When it is determined that the position of the first posture positioning feature in the fourth target image is not a second predetermined position, the current measurement posture is adjusted to obtain a new fourth target image until the position of the first posture positioning feature in the new fourth target image is the second predetermined position. When it is determined that the position of the first posture positioning feature in the new fourth target image is the second predetermined position, it is determined that the current measurement posture is the target measurement posture.

According to embodiments of the present disclosure, the device 1400 of measuring the tissue element further includes a fourth image acquisition portion arranged on the measured object, the measurement probe 1450, the fixing portion 1460 or other objects. The fourth image acquisition portion is used to acquire the fourth target image.

According to embodiments of the present disclosure, the fourth image acquisition portion, the third image acquisition portion 1510, the first image acquisition portion 1490 and the second image acquisition portion may be different, partially the same, or completely the same.

According to embodiments of the present disclosure, when it is determined that the fourth image acquisition portion is arranged on the measurement probe 1450, the first posture positioning feature is not arranged on the measurement probe 1450. When it is determined that the fourth image acquisition portion is arranged on the fixing portion 1460, the first posture positioning feature is not arranged on the fixing portion 1460.

According to embodiments of the present disclosure, the device 1400 of measuring the tissue element further includes a third determination module and an adjustment module.

The third determination module is used to determine a second posture positioning feature in response to the current measurement posture being not the target measurement posture if the measurement probe 1450 is arranged at the position corresponding to the measurement region. The adjustment module is used to adjust the current measurement posture to the target measurement posture according to the second posture positioning feature.

According to embodiments of the present disclosure, the adjustment module includes a first obtaining unit, a second adjustment unit, and a second determination unit.

The first obtaining unit is used to obtain a third projection feature. The second adjustment unit is used to adjust, when it is determined that the second posture positioning feature is not matched with the third projection feature, the current measurement posture until the second posture positioning feature is matched with the third projection feature. The second determination unit is used to determine that the current measurement posture is the target measurement posture when it is determined that the second posture positioning feature is matched with the third projection feature.

According to embodiments of the present disclosure, the device 1400 of measuring the tissue element further includes a second posture positioning portion arranged on the measured object, the measurement probe 1450, the fixing portion 1460 or other objects. The second posture positioning portion is used to project the third projection feature.

According to embodiments of the present disclosure, when it is determined that the second posture positioning portion is arranged on the measurement probe 1450, the second posture positioning feature is not arranged on the measurement probe 1450 and the fixing portion 1460. When it is determined that the second posture positioning portion is arranged on the fixing portion 1460, the second posture positioning feature is not arranged on the measurement probe 1450 and the fixing portion 1460.

According to embodiments of the present disclosure, the second posture positioning portion is the same as or different from the first posture positioning portion 1500.

According to embodiments of the present disclosure, the second posture positioning portion includes a third laser.

According to embodiments of the present disclosure, the third laser may project a light spot having a predetermined shape, so as to form the third projection feature.

According to embodiments of the present disclosure, the adjustment module includes a second obtaining unit, a third obtaining unit, a third adjustment unit, and a third determination unit.

The second obtaining unit is used to obtain a fifth target image. The third obtaining unit is used to obtain a third template image, where the third template image contains the second posture positioning feature. The third adjustment unit is used to adjust, when it is determined that the fifth target image is not matched with the third template image, the current measurement posture to obtain a new fifth target image until the new fifth target image is matched with the third template image. The third determination unit is used to determine that the current measurement posture is the target measurement posture when it is determined that the new fifth target image is matched with the third template image.

According to embodiments of the present disclosure, the device 1400 of measuring the tissue element further includes a fifth image acquisition portion arranged on the measured object, the measurement probe 1450, the fixing portion 1460 or other objects. The fifth image acquisition portion is used to acquire the fifth target image.

According to embodiments of the present disclosure, the adjustment module includes a fourth obtaining unit, a fourth adjustment unit, and a fourth determination unit.

The fourth obtaining unit is used to obtain a sixth target image, where the sixth target image contains the second posture positioning feature. The fourth adjustment unit is used to adjust, when it is determined that the position of the second posture positioning feature in the sixth target image is not a third predetermined position, the current measurement posture to obtain a new sixth target image until the position of the second posture positioning feature in the new sixth target image is the third predetermined position. The fourth determination unit is used to determine that the current measurement posture is the target measurement posture when it is determined that the position of the second posture positioning feature in the new sixth target image is the third predetermined position.

According to embodiments of the present disclosure, the device 1400 of measuring the tissue element further includes a sixth image acquisition portion arranged on the measured object, the measurement probe 1450, the fixing portion 1460 or other objects. The sixth image acquisition portion is used to acquire the sixth target image.

According to embodiments of the present disclosure, when it is determined that the sixth image acquisition portion is arranged on the measurement probe 1450, the second posture positioning feature is not arranged on the measurement probe 1450 and the fixing portion 1460. When it is determined that the sixth image acquisition portion is arranged on the fixing portion 1460, the second posture positioning feature is not arranged on the measurement probe 1450 and the fixing portion 1460.

According to embodiments of the present disclosure, the sixth image acquisition portion, the fifth image acquisition portion, the fourth image acquisition portion, the third image acquisition portion 1510, the first image acquisition portion 1490 and the second image acquisition portion may be different, partially the same, or completely the same.

According to embodiments of the present disclosure, when the measurement region and the measurement posture are positioned using an optical method, the region positioning portion 1480, the first posture positioning portion 1500 and the second posture positioning portion may be all the same, partially the same, or completely different. The above-mentioned partially the same means that two of the above three structures are the same. If the three structures are all the same, it may indicate that a same structure may be used to generate the first projection feature, the second projection feature and the third projection feature. The above method may simplify the complexity of the positioning structure.

When the measurement region and the measurement posture are positioned using an image matching method, the first image acquisition portion 1490, the third image acquisition portion 1510 and the fifth image acquisition portion may be all the same, partially the same or completely different. The above-mentioned partially the same means that two of the above three structures are the same. If the three structures are all the same, it may indicate that a same structure may be used to generate the first target image, the third target image and the fifth target image. The above method may simplify the complexity of the positioning structure.

When the measurement region and the measurement posture are positioned using an imaging method, the second image acquisition portion, the fourth image acquisition portion and the sixth image acquisition portion may be all the same, partially the same or completely different. The above-mentioned partially the same means that two of the above three structures are the same. If the three structures are all the same, it may indicate that a same structure may be used to generate the second target image, the fourth target image and the sixth target image. The above method may simplify the complexity of the positioning structure. A description will be given below by taking the optical method as an example.

The following description is for a case that the region positioning portion 1480, the first posture positioning portion 1500 and the second posture positioning portion are the same structure, the second posture positioning feature is completely identical to the region positioning feature and is partially identical to the first posture positioning feature, and the measurement region is a forearm extensor side.

FIG. 25 schematically shows a schematic diagram of positioning the measurement posture and the measurement region according to embodiments of the present disclosure. The region positioning portion 1480, the first posture positioning portion 1500 and the second posture positioning portion all include a laser 1 and a laser 2. The laser 1 and the laser 2 are arranged on the measurement probe 1450.

When a first positioning of the measurement posture is performed, the measurement probe 1450 is arranged on a base, and the position of the measurement probe 1450 is fixed before the first positioning of measurement posture is completed. The current measurement posture is adjusted according to the first posture positioning feature and the second projection feature until the first posture positioning feature is matched with the second projection feature. When the two are matched, it may indicate that the first positioning of the measurement posture is completed.

When the positioning of the measurement region is performed, the measurement probe 1450 is arranged on the measured object. The position of the measurement probe 1450 is adjusted according to the region positioning feature and the first projection feature until the region positioning feature is matched with the first projection feature. When the two are matched, it may indicate that the positioning of the measurement region is completed.

After the measurement probe 1450 is arranged on the measured object, if the current measurement posture is not the target posture, it is needed to perform a second positioning of the measurement posture before the measurement is performed. The current measurement posture is adjusted according to the second posture positioning feature and the third projection feature until the second posture positioning feature is matched with the third projection feature. When the two are matched, it may indicate that the second positioning of the measurement posture is completed.

The following description is for a case that the region positioning portion 1480, the first posture positioning portion 1500 and the second posture positioning portion are the same structure, the region positioning feature is completely identical to the second posture positioning feature and partially identical to the first posture positioning feature, and the measurement region is a forearm extensor side.

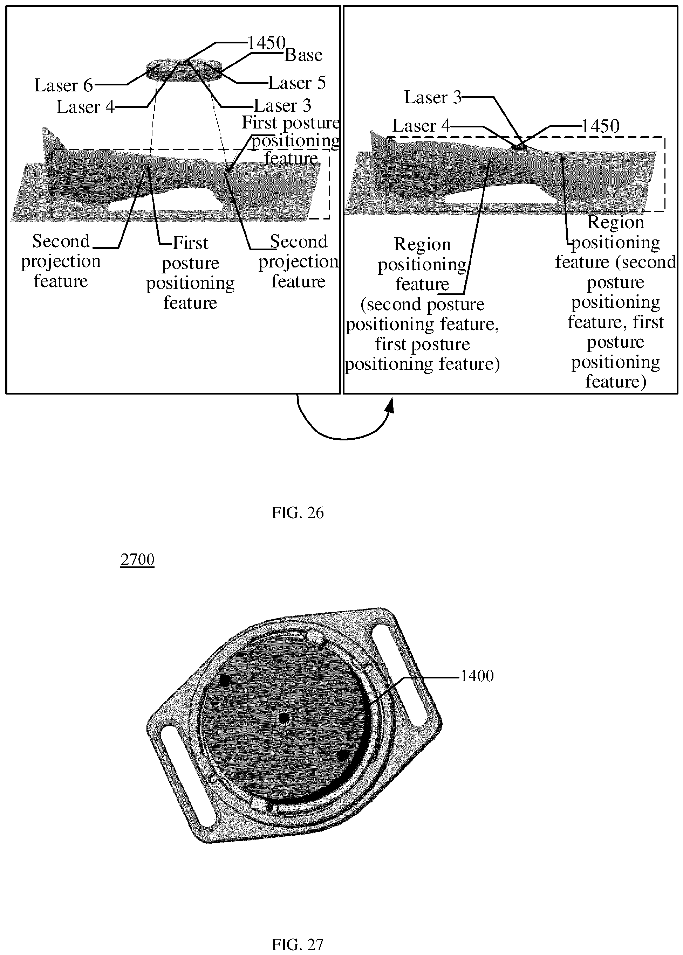

FIG. 26 schematically shows another schematic diagram of positioning the measurement posture and the measurement region according to embodiments of the present disclosure. In FIG. 26, both the region positioning portion 1480 and the second posture positioning portion include a laser 3 and a laser 4. The first posture positioning portion 1500 includes a laser 5 and a laser 6. The laser 3 and the laser 4 are arranged on the measurement probe 1450. The laser 5 and the laser 6 are arranged on the base.

According to embodiments of the present disclosure, the device 1400 of measuring the tissue element further includes a prompt module.

The prompt module is used to generate a prompt information, which is used to prompt a completion of the positioning of the measurement posture and/or the positioning of the measurement region. A form of the prompt information includes at least one selected from an image, a speech, or a vibration.

According to embodiments of the present disclosure, the measurement module includes a light source unit, an acquisition unit, and a seventh determination unit.

The light source unit is used to illuminate the measurement region by incident light having at least one predetermined wavelength, where each beam of the incident light is incident on an incident position to form at least one beam of exit light exited from at least one exit position on the measurement region. The acquisition unit is used to obtain a light intensity value corresponding to each beam of the exit light acquired by the measurement probe 1450, so as to obtain T output light intensities, where the measurement probe 1450 includes M photosensitive surfaces, and each output light intensity is obtained by processing the light intensity value of the exit light acquired by one or more photosensitive surfaces, 1≤T≤M. The seventh determination unit is used to determine a concentration of the measured tissue element according to at least one output light intensity corresponding to the at least one predetermined wavelength.

According to embodiments of the present disclosure, the seventh determination unit is used to determine, for each predetermined wavelength in the at least one predetermined wavelength, a first output light intensity and a second output light intensity from at least two output light intensities corresponding to the predetermined wavelength. A differential processing is performed on the first output light intensity and the second output light intensity corresponding to the predetermined wavelength to obtain a differential signal. The concentration of the measured tissue element is determined according to the differential signal corresponding to each predetermined wavelength.

According to embodiments of the present disclosure, each photosensitive surface may acquire the light intensity value of the exit light exited from the exit position within the predetermined anti-jitter range corresponding to the photosensitive surface.

According to embodiments of the present disclosure, a ratio of an average optical path of the exit light received by each photosensitive surface in a target tissue layer to a total optical path is greater than or equal to a ratio threshold. The total optical path is a total distance that the exit light travels in the measurement region.

According to embodiments of the present disclosure, a total area of the homogeneous photosensitive surface is determined according to a tissue structure feature in the measurement region. The homogeneous photosensitive surface includes one or more photosensitive surfaces, and the homogeneous photosensitive surface is used to output one output light intensity.

According to embodiments of the present disclosure, a ratio of the area of each photosensitive surface to a circumference of the photosensitive surface is greater than or equal to a ratio threshold.

According to embodiments of the present disclosure, the ratio threshold is greater than or equal to 0.04 mm.

According to embodiments of the present disclosure, the photosensitive surface is in contact or non-contact with a surface of the measurement region.

According to embodiments of the present disclosure, a distance between the photosensitive surface and the surface of the measurement region is less than or equal to a distance threshold, and the efficiency of the photosensitive surface receiving the exit light is greater than or equal to an efficiency threshold.

Any number of the modules and units according to embodiments of the present disclosure, or at least part of functions of any number of them may be implemented in one module. Any one or more of the modules and units according to embodiments of the present disclosure may be split into a plurality of modules for implementation. Any one or more of the modules and units according to embodiments of the present disclosure may be implemented at least partially as a hardware circuit, such as a field programmable gate array (FPGA), a programmable logic array (PLA), a system on a chip, a system on a substrate, a system on a package, an application specific integrated circuit (ASIC), or may be implemented by hardware or firmware in any other rational manner of integrating or encapsulating the circuit, or may be implemented by any one of three implementation modes of software, hardware and firmware or an appropriate combination thereof. Alternatively, one or more of the modules and units according to embodiments of the present disclosure may be at least partially implemented as a computer program module that may performs the corresponding functions when executed.

For example, any number of the measurement module may be combined into one module/unit for implementation, or any one of the modules/units may be split into a plurality of modules/units. Alternatively, at least part of the functions of one or more of these modules/units may be combined with at least part of the functions of other modules/units and implemented in one module/unit. According to embodiments of the present disclosure, at least one of the measurement module may be implemented at least partially as a hardware circuit, such as a field programmable gate array (FPGA), a programmable logic array (PLA), a system on a chip, a system on a substrate, a system on a package, an application specific integrated circuit (ASIC), or may be implemented by hardware or firmware in any other rational manner of integrating or encapsulating the circuit, or may be implemented by any one of the three implementation modes of software, hardware and firmware or an appropriate combination thereof. Alternatively, at least one of the measurement module may be at least partially implemented as a computer program module that may perform the corresponding functions when executed.

It should be noted that the device of measuring the tissue element in embodiments of the present disclosure corresponds to the description of the method of measuring the tissue element in embodiments of the present disclosure. For the description of the device of measuring the tissue element, reference may be specifically made to the description of the method of measuring the tissue element, which will not be repeated here.

FIG. 27 schematically shows a schematic diagram of a wearable apparatus according to embodiments of the present disclosure. A wearable apparatus 2700 shown in FIG. 27 is just an example, and should not impose any limitation on a function and a scope of use of the present disclosure.

As shown in FIG. 27, the wearable apparatus 2700 includes the device 1400 of measuring the tissue element.

According to the technical solutions of embodiments of the present disclosure, by determining the positioning feature, determining the measurement region meeting the reproducibility of the measurement condition according to the positioning feature, arranging the measurement probe 1450 at the position corresponding to the measurement region, and performing a tissue element measurement by using the measurement probe 1450, the measurement region may be positioned accurately, and then the reproducibility of the measurement condition may be effectively controlled.

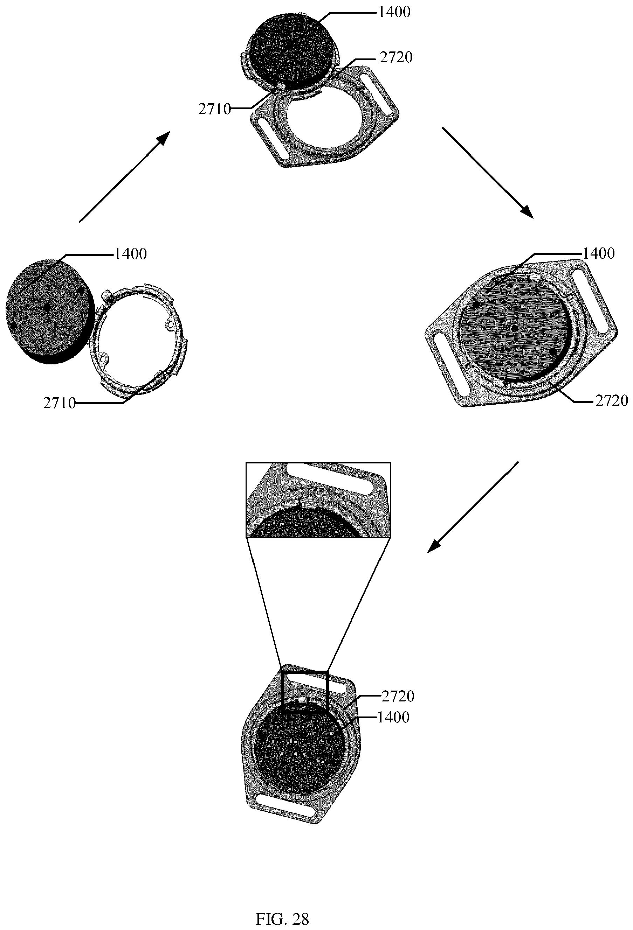

As shown in FIG. 28, according to embodiments of the present disclosure, the wearable apparatus 2700 further includes a buckle portion 2710 and a body 2720. The buckle portion 2710 and the body 2720 are used to cooperate to fix the device 1400 of measuring the tissue element.

According to embodiments of the present disclosure, FIG. 28 schematically shows a schematic diagram of an assembly process of the wearable apparatus according to embodiments of the present disclosure.

According to embodiments of the present disclosure, a mass of the wearable apparatus 2700 is less than or equal to a mass threshold, so that a movement pattern of the wearable apparatus 2700 is consistent with a skin jitter pattern at the measurement region.

According to embodiments of the present disclosure, in order to improve the reliability of the measurement result, the wearable apparatus 2700 may have a small mass, so that the wearable apparatus 2700 may follow the skin jitter at the measurement region when the wearable apparatus 2700 is arranged at a position corresponding to the measurement region, that is, the movement pattern of the wearable apparatus 2700 may be consistent with the skin jitter pattern at the measurement region, and then the average optical path of the exit light received by the measurement probe 1450 may be maintained within the predetermined optical path range during the skin jitter process. The average optical path of the exit light received by the measurement probe 1450 may be maintained within the predetermined optical path range during the skin jitter process of the measurement region because a relative position of the measurement probe 1450 on the measurement region may be kept unchanged or substantially unchanged if the wearable apparatus 2700 may follow the skin jitter at the measurement region, and then the measurement probe 1450 may receive the exit light exited from a fixed exit position. The fixed exit position here means an exit position that remains unchanged or substantially unchanged relative to the measurement region. Furthermore, during the skin jitter process of the measurement region, the incident position of the incident light may remain unchanged or substantially unchanged relative to the measurement region. In a case that the incident position of the incident light and the exit position of the exit light are determined, it is possible to ensure as much as possible that the average optical path of the exit light remains unchanged.

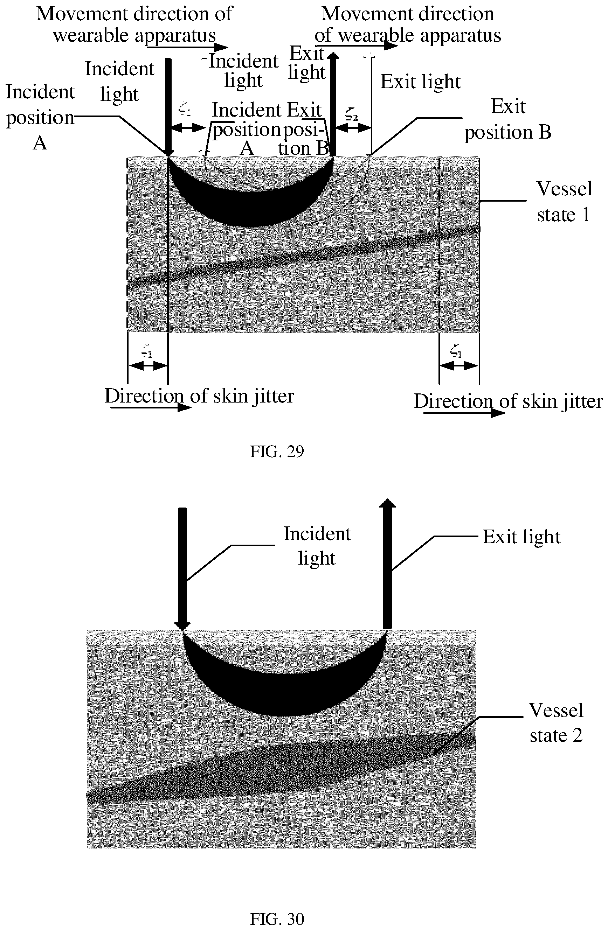

Exemplarily, FIG. 29 schematically shows a schematic diagram of maintaining the average optical path of the exit light received by the measurement probe within a predetermined optical path range during a skin jitter process in a case that the wearable apparatus is consistent with a skin jitter pattern according to embodiments of the present disclosure. During the skin jitter process, the measurement probe 1450 (not shown in FIG. 29) may stably receive the exit light that is exited from the exit position B on the measurement region after the incident light is incident on the incident position A on the measurement region. A movement amplitude of the skin is represented by ζ₁, and a movement amplitude of the measurement probe 1450 is represented by ζ₂, ζ₁=ζ₂.

According to embodiments of the present disclosure, the wearable apparatus 2700 causes the movement amplitude of the skin at the measurement region to be less than or equal to a movement amplitude threshold.

According to embodiments of the present disclosure, in order to improve the reliability of the measurement result, the wearable apparatus 2700 may have a large mass, so that when the wearable apparatus 2700 is arranged at a position corresponding to the measurement region, it may press the skin jitter at the measurement region, that is, the movement amplitude of the skin at the measurement region is less than or equal to a movement amplitude threshold, and then the average optical path of the exit light received by the measurement probe 1450 may be maintained within a predetermined optical path range during the skin jitter process. The average optical path of the exit light received by the measurement probe 1450 may be maintained within the predetermined optical path range during the skin jitter process at the measurement region because a relative position of the measurement probe 1450 on the measurement region may be kept unchanged or substantially unchanged if the wearable apparatus 2700 may press the skin jitter at the measurement region, and then the measurement probe 1450 may receive the exit light exited from a fixed exit position. Furthermore, during the skin jitter process of the measurement region, the incident position of the incident light may remain unchanged or substantially unchanged relative to the measurement region. In a case that the incident position of the incident light and the exit position of the exit light are determined, it is possible to ensure as much as possible that the average optical path of the exit light remains unchanged.

Exemplarily, FIG. 30 schematically shows a schematic diagram of maintaining the average optical path of the exit light received by the measurement probe within a predetermined optical path range during a skin jitter process in a case that the wearable apparatus causes a movement amplitude of a skin at the measurement region to be less than or equal to a movement amplitude threshold according to embodiments of the present disclosure. In FIG. 30, the movement amplitude of the skin at the measurement region is close to zero.

According to embodiments of the present disclosure, for the specific description of the device of measuring the tissue element, reference may be made to the corresponding part above, and details will not be repeated here. In addition, the device of measuring the tissue element includes a processor which may perform various appropriate actions and processes according to a program stored in a read only memory (ROM) or a program loaded from a storage portion into a random access memory (RAM). The processor may include, for example, a general-purpose microprocessor (for example, CPU), an instruction set processor and/or a related chipset and/or a special-purpose microprocessor (for example, an application specific integrated circuit (ASIC)), and the like. The processor may further include an on-board memory for caching purposes. The processor may include a single processing unit or a plurality of processing units for performing different actions of the method flow according to embodiments of the present disclosure.

Various programs and data required for the operation of the tissue element measurement device are stored in the RAM. The processor, the ROM and the RAM are connected to each other through a bus. The processor performs various operations of the method flow according to embodiments of the present disclosure by executing the programs in the ROM and/or the RAM. It should be noted that the program may also be stored in one or more memories other than the ROM and the RAM. The processor may also perform various operations of the method flow according to embodiments of the present disclosure by executing the programs stored in the one or more memories.

According to embodiments of the present disclosure, the wearable apparatus may further include an input/output (I/O) interface which is also connected to the bus. The wearable apparatus may further include one or more of the following components connected to the I/O interface: an input part including a keyboard, a mouse, etc.; an output part including a cathode ray tube (CRT), a liquid crystal display (LCD), etc. and a speaker, etc.; a storage part including a hard disk, etc.; and a communication part including a network interface card such as a LAN card, a modem, and the like. The communication part performs communication processing via a network such as the Internet. A drive is also connected to the I/O interface as required. A removable medium, such as a magnetic disk, an optical disk, a magneto-optical disk, a semiconductor memory, and the like, is installed on the drive as required, so that the computer program read therefrom is installed into the storage part.

The present disclosure further provides a computer-readable storage medium, which may be included in the apparatus/device/system described in the above embodiments; or exist alone without being assembled into the apparatus/device/system. The above-mentioned computer-readable storage medium carries one or more programs that perform the methods according to embodiments of the present disclosure when being executed.

According to embodiments of the present disclosure, the computer-readable storage medium may be a non-transitory computer-readable storage medium, for example, may include but not limited to: a portable computer disk, a hard disk, a random access memory (RAM), a read-only memory (ROM), an erasable programmable read-only memory (EPROM) or flash memory, a portable compact disk read-only memory (CD-ROM), an optical storage device, a magnetic storage device, or any suitable combination of the above. In the present disclosure, the computer-readable storage medium may be any tangible medium that contains or stores programs that may be used by or in combination with an instruction execution system, apparatus or device.

For example, according to embodiments of the present disclosure, the computer-readable storage medium may include the above-mentioned ROM and/or RAM and/or one or more memories other than the ROM and RAM.

Embodiments of the present disclosure further include a computer program product, which contains a computer program. The computer program contains program code for performing the method provided by embodiments of the present disclosure.

When the computer program is executed by the processor, the above-mentioned functions defined in the system/device of embodiments of the present disclosure are performed. According to embodiments of the present disclosure, the above-described systems, devices, modules, units, etc. may be implemented by computer program modules.

In an embodiment, the computer program may rely on a tangible storage medium such as an optical storage device and a magnetic storage device. In another embodiment, the computer program may also be transmitted and distributed in the form of signals on a network medium, downloaded and installed through the communication part, and/or installed from the removable medium. The program code contained in the computer program may be transmitted by any suitable medium, including but not limited to a wireless one, a wired one, or any suitable combination of the above.

According to embodiments of the present disclosure, the program code for executing the computer programs provided by embodiments of the present disclosure may be written in any combination of one or more programming languages. In particular, these computing programs may be implemented using high-level procedures and/or object-oriented programming languages, and/or assembly/machine languages. Programming languages include, but are not limited to, Java, C++, Python, "C" language or similar programming languages. The program code may be completely executed on the user computing apparatus, partially executed on the user device, partially executed on the remote computing apparatus, or completely executed on the remote computing apparatus or server. In a case of involving a remote computing apparatus, the remote computing apparatus may be connected to a user computing apparatus through any kind of network, including a local area network (LAN) or a wide area networks (WAN), or may be connected to an external computing apparatus (e.g., through the Internet using an Internet service provider).

The flowcharts and block diagrams in the accompanying drawings illustrate the possible architecture, functions, and operations of the system, method, and computer program product according to various embodiments of the present disclosure. In this regard, each block in the flowcharts or block diagrams may represent a part of a module, a program segment, or a code, which part includes one or more executable instructions for implementing the specified logical function. It should be further noted that, in some alternative implementations, the functions noted in the blocks may also occur in a different order from that noted in the accompanying drawings. For example, two blocks shown in succession may actually be executed substantially in parallel, or they may sometimes be executed in a reverse order, depending on the functions involved. It should be further noted that each block in the block diagrams or flowcharts, and the combination of blocks in the block diagrams or flowcharts, may be implemented by a dedicated hardware-based system that performs the specified functions or operations, or may be implemented by a combination of dedicated hardware and computer instructions. Those skilled in the art may understand that the various embodiments of the present disclosure and/or the features described in the claims may be combined in various ways, even if such combinations are not explicitly described in the present disclosure. In particular, the various embodiments of the present disclosure and/or the features described in the claims may be combined in various ways without departing from the spirit and teachings of the present disclosure. All these combinations fall within the scope of the present disclosure.

Embodiments of the present disclosure have been described above. However, these embodiments are for illustrative purposes only, and are not intended to limit the scope of the present disclosure. Although the various embodiments have been described separately above, this does not mean that measures in the respective embodiments may not be used in combination advantageously. The scope of the present disclosure is defined by the appended claims and their equivalents. Those skilled in the art may make various substitutions and modifications without departing from the scope of the present disclosure, and these substitutions and modifications should all fall within the scope of the present disclosure.

## Claims

1. A method of measuring a tissue element, comprising:
determining a positioning feature;
determining a measurement region according to the positioning feature, wherein the measurement region meets a reproducibility of a measurement condition;
arranging the measurement probe at a position corresponding to the measurement region; and
performing a tissue element measurement by using the measurement probe.

2. The method according to claim 1, wherein the positioning feature comprises a first posture positioning feature and a region positioning feature;
the determining a measurement region according to the positioning feature comprises:
adjusting a current measurement posture of a measured object to a target measurement posture according to the first posture positioning feature, wherein the target measurement posture meets the reproducibility of the measurement condition; and
determining the measurement region according to the region positioning feature, in response to the current measurement posture being the target measurement posture.

3. The method according to claim 2, wherein the arranging a measurement probe at a position corresponding to the measurement region comprises:
arranging the measurement probe at the position corresponding to the measurement region by a fixing portion, wherein the fixing portion is integrated with, partially separated from or completely separated from the measurement probe.

4. The method according to claim 3, wherein the fixing portion comprises a fixing seat and a first fitting part;
the arranging the measurement probe at the position corresponding to the measurement region by the fixing portion comprises:
arranging the fixing seat at the position corresponding to the measurement region by the first fitting part; and
arranging the measurement probe on the fixing seat.

5. The method according to claim 4, wherein a skin state of a skin at the measurement region meets a first predetermined condition in a process of arranging the fixing seat at the position corresponding to the measurement region by the first fitting part.

6. The method according to claim 4, wherein a skin state of a skin at the measurement region meets a second predetermined condition in a process of arranging the measurement probe on the fixing seat.

7. The method according to claim 4, wherein the measurement probe is non-movable on the fixing seat.

8. The method according to claim 3, wherein the fixing portion comprises a second fitting part;
the arranging the measurement probe at the position corresponding to the measurement region by the fixing portion comprises:
arranging the measurement probe at the position corresponding to the measurement region by the second fitting part.

9. The method according to claim 8, wherein a skin state of a skin at the measurement region meets a third predetermined condition in a process of arranging the measurement probe at the position corresponding to the measurement region by the second fitting part.

10. The method according to claim 3, wherein the determining the measurement region according to the region positioning feature comprises:
obtaining a first projection feature;
adjusting, in response to a determination that the region positioning feature is not matched with the first projection feature, a position of the measurement probe and/or the fixing portion until the region positioning feature is matched with the first projection feature; and
determining a region corresponding to the measurement probe and/or the fixing portion as the measurement region in response to a determination that the region positioning feature is matched with the first projection feature.

11. The method according to claim 3, wherein the determining the measurement region according to the region positioning feature comprises:
obtaining a first target image;
obtaining a first template image, wherein the first template image contains the region positioning feature;
adjusting, in response to a determination that the first target image is not matched with the first template image, a position of the measurement probe and/or the fixing portion to obtain a new first target image until the new first target image is matched with the first template image; and
determining a region corresponding to the measurement probe and/or the fixing portion as the measurement region in response to a determination that the first target image is matched with the first template image.

12. The method according to claim 3, wherein the determining the measurement region according to the region positioning feature comprises:
obtaining a second target image, wherein the second target image contains the region positioning feature;
adjusting, in response to a determination that a position of the region positioning feature in the second target image is not a first predetermined position, a position of the measurement probe and the fixing portion to obtain a new second target image until the position of the region positioning feature in the new second target image is the first predetermined position; and
determining a region corresponding to the measurement probe and the fixing portion as the measurement region in response to a determination that the position of the region positioning feature in the new second target image is the first predetermined position.

13. The method according to claim 3, wherein the adjusting a current measurement posture of a measured object to a target measurement posture according to the first posture positioning feature comprises:
obtaining a second projection feature;
adjusting, in response to a determination that the first posture positioning feature is not matched with the second projection feature, the current measurement posture until the first posture positioning feature is matched with the second projection feature; and
determining that the current measurement posture is the target measurement posture, in response to a determination that the first posture positioning feature is matched with the second projection feature.

14. The method according to claim 3, wherein the adjusting a current measurement posture of a measured object to a target measurement posture according to the first posture positioning feature comprises:
obtaining a third target image;
obtaining a second template image, wherein the second template image contains the first posture positioning feature;
adjusting, in response to a determination that the third target image is not matched with the second template image, the current measurement posture to obtain a new third target image until the new third target image is matched with the second template image; and
determining that the current measurement posture is the target measurement posture, in response to a determination that the new third target image is matched with the second template image.

15. The method according to claim 3, wherein the adjusting a current measurement posture of a measured object to a target measurement posture according to the first posture positioning feature comprises:
obtaining a fourth target image, wherein the fourth target image contains the first posture positioning feature;
adjusting, in response to a determination that a position of the first posture positioning feature in the fourth target image is not a second predetermined position, the current measurement posture to obtain a new fourth target image until the position of the first posture positioning feature in the new fourth target image is the second predetermined position; and
determining that the current measurement posture is the target measurement posture, in response to a determination that the position of the first posture positioning feature in the new fourth target image is the second predetermined position.

16. The method according to claim 3, further comprising:
determining a second posture positioning feature in response to a determination that the current measurement posture is not the target measurement posture, if the measurement probe is arranged at the position corresponding to the measurement region; and
adjusting the current measurement posture to the target measurement posture according to the second posture positioning feature.

17. The method according to claim 16, wherein the adjusting the current measurement posture to the target measurement posture according to the second posture positioning feature comprises:
obtaining a third projection feature;
adjusting, in response to a determination that the second posture positioning feature is not matched with the third projection feature, the current measurement posture until the second posture positioning feature is matched with the third projection feature; and
determining that the current measurement posture is the target measurement posture, in response to a determination that the second posture positioning feature is matched with the third projection feature.

18. The method according to claim 16, wherein the adjusting the current measurement posture to the target measurement posture according to the second posture positioning feature comprises:
obtaining a fifth target image;
obtaining a third template image, wherein the third template image contains the second posture positioning feature;
adjusting, in response to a determination that the fifth target image is not matched with the third template image, the current measurement posture to obtain a new fifth target image until the new fifth target image is matched with the third template image; and
determining that the current measurement posture is the target measurement posture, in response to a determination that the new fifth target image is matched with the third template image.

19. The method according to claim 16, wherein the adjusting the current measurement posture to the target measurement posture according to the second posture positioning feature comprises:
obtaining a sixth target image, wherein the sixth target image contains the second posture positioning feature;
adjusting, in response to a determination that a position of the second posture positioning feature in the sixth target image is not a third predetermined position, the current measurement posture to obtain a new sixth target image until the position of the second posture positioning feature in the new sixth target image is the third predetermined position; and
determining that the current measurement posture is the target measurement posture, in response to a determination that the position of the second posture positioning feature in the new sixth target image is the third predetermined position.

20. The method according to claim 16, further comprising:
generating a prompt information, wherein the prompt information is configured to prompt a completion of a positioning of the measurement posture and/or a positioning of the measurement region, and a form of the prompt information comprises at least one selected from an image, a speech, or a vibration.

21. The method according to claim 4, further comprising:
arranging the measurement probe on the fixing seat in response to a determination that the fixing seat is arranged at the position corresponding to the measurement region and the measurement probe is not arranged on the fixing portion; or
arranging the fixing seat at the position corresponding to the measurement region by the first fitting part and arranging the measurement probe on the fixing seat, in response to a determination that the fixing seat is not arranged at the position corresponding to the measurement region.

22. The method according to claim 8, further comprising:
arranging the measurement probe at a position corresponding to the measurement region by the second fitting part, in response to a determination that the measurement probe is not arranged at the position corresponding to the measurement region.

23. The method according to claim 1, wherein the performing a tissue element measurement by using the measurement probe comprises:
irradiating a measurement region with incident light having at least one predetermined wavelength, wherein each beam of the incident light is incident on an incident position to form at least one beam of exit light exited from at least one exit position on the measurement region;
obtaining a light intensity value corresponding to each beam of the exit light acquired by the measurement probe, so as to obtain T output light intensities, wherein the measurement probe comprises M photosensitive surfaces, each of the T output light intensities is obtained by processing the light intensity value of the exit light acquired by one or more of the M photosensitive surfaces, 1<T <M; and
determining a concentration of a measured tissue element according to at least one output light intensity corresponding to the at least one predetermined wavelength.

24. The method according to claim 23, wherein the determining a concentration of a measured tissue element according to at least one output light intensity corresponding to the at least one predetermined wavelength comprises:
determining, for each predetermined wavelength in the at least one predetermined wavelength, a first output light intensity and a second output light intensity from at least two output light intensities corresponding to the predetermined wavelength;
performing a differential processing on the first output light intensity and the second output light intensity corresponding to the predetermined wavelength, so as to obtain a differential signal; and
determining the concentration of the measured tissue element according to the differential signal corresponding to each predetermined wavelength.

25. The method according to claim 23, wherein each photosensitive surface is configured to acquire the light intensity value of the exit light exited from the exit position within a predetermined anti-jitter range corresponding to the photosensitive surface.

26. The method according to claim 23, wherein a ratio of an average optical path of the exit light received by each photosensitive surface in a target tissue layer to a total optical path is greater than or equal to a ratio threshold, and the total optical path is a total distance that the exit light travels in the measurement region.

27. The method according to claim 23, further comprising:
determining a total area of the homogeneous photosensitive surface according to a tissue structure feature in the measurement region, wherein the homogeneous photosensitive surface comprises one or more photosensitive surfaces, and the homogeneous photosensitive surface is configured to output one output light intensity.

28. The method according to claim 23, wherein a ratio of an area of each photosensitive surface to a circumference of the photosensitive surface is greater than or equal to a ratio threshold.

29. The method according to claim 28, wherein the ratio threshold is greater than or equal to 0.04 mm.

30. The method according to claim 25 or 26, wherein the photosensitive surface is in contact or non-contact with a surface of the measurement region.

31. The method according to claim 30, wherein a distance between the photosensitive surface and the surface of the measurement region is less than or equal to a distance threshold, and an efficiency of the photosensitive surface receiving the exit light is greater than or equal to an efficiency threshold.

32. A device of measuring a tissue element, comprising:
a first determination module configured to determine a positioning feature;
a second determination module configured to determine a measurement region according to the positioning feature, wherein the measurement region meets a reproducibility of a measurement condition;
an arrangement module configured to arrange a measurement probe at a position corresponding to the measurement region; and
a measurement module configured to perform a tissue element measurement by using the measurement probe.

33. The device according to claim 32, wherein the positioning feature comprises a first posture positioning feature and a region positioning feature;
the second determination module comprises:
a first adjustment unit configured to adjust a current measurement posture of a measured object to a target measurement posture according to the first posture positioning feature, wherein the target measurement posture meets the reproducibility of the measurement condition; and
a first determination unit configured to determine the measurement region according to the region positioning feature, in response to a determination that the current measurement posture is the target measurement posture.

34. The device according to claim 33, further comprising a fixing portion configured to arrange the measurement probe at the position corresponding to the measurement region, wherein the fixing portion is integrated with, partially separated from or completely separated from the measurement probe.

35. The device according to claim 34, wherein the fixing portion comprises a fixing seat and a first fitting part;
the first fitting part is configured to arrange the fixing seat at the position corresponding to the measurement region; and
the fixing seat is configured to fix the measurement probe.

36. The device according to claim 35, wherein a hardness of the first fitting part comprises a first hardness and a second hardness, the first hardness is less than the second hardness, the first hardness is a corresponding hardness in a process of fixing the fixing seat by the first fitting part, and the second hardness is a corresponding hardness after the fixing seat is fixed by the first fitting part.

37. The device according to claim 36, wherein the first fitting part comprises a first Velcro or a first elastic belt.

38. The device according to claim 35, wherein a surface of the first fitting part is provided with a hole.

39. The device according to claim 36, wherein the hardness of the first fitting part is greater than or equal to a first hardness threshold and less than or equal to a second hardness threshold.

40. The device according to claim 36, further comprising a magnetic portion, wherein the first fitting part is entirely or partially a metal hinge, and the magnetic portion fits the first fitting part to fix the fixing seat.

41. The device according to claim 35, wherein the measurement probe is fixed on the fixing seat by at least one method selected from:
the measurement probe being fixed on the fixing seat by an adhesive tape;
the measurement probe being fixed on the fixing seat by a fastener;
the measurement probe being fixed on the fixing seat by a magnetic force; or
a friction coefficient between the measurement probe and the fixing seat being greater than or equal to a friction coefficient threshold.

42. The device according to claim 34, wherein the fixing portion comprises a second fitting part configured to arrange the measurement probe at the position corresponding to the measurement region.

43. The device according to claim 42, wherein a hardness of the second fitting part comprises a third hardness and a fourth hardness, the third hardness is less than the fourth hardness, the third hardness is a corresponding hardness in a process of fixing the measurement probe by the second fitting part, and the fourth hardness is a corresponding hardness after the measurement probe is fixed by the second fitting part.

44. The device according to claim 43, wherein the second fitting part comprises a second Velcro or a second elastic belt.

45. The device according to claim 42, wherein a surface of the second fitting part is provided with a hole.

46. The device according to claim 43, wherein the hardness of the second fitting part is greater than or equal to a third hardness threshold and less than or equal to a fourth hardness threshold.

47. The device according to claim 34, wherein the first determination unit is configured to:
obtain a first projection feature;
adjust, in response to a determination that the region positioning feature is not matched with the first projection feature, a position of the measurement probe and/or the fixing portion until the region positioning feature is matched with the first projection feature; and
determine a region corresponding to the measurement probe and/or the fixing portion as the measurement region in response to a determination that the region positioning feature is matched with the first projection feature.

48. The device according to claim 46, further comprising a region positioning portion arranged on the measured object, the measurement probe, the fixing portion or other objects, wherein the region positioning portion is configured to project the first projection feature.

49. The device according to claim 48, wherein the region positioning feature is not arranged on the measurement probe when it is determined that the region positioning portion is arranged on the measurement probe; and
wherein the region positioning feature is not arranged on the fixing portion when it is determined that the region positioning portion is arranged on the fixing portion.

50. The device according to claim 48, wherein the region positioning portion comprises a first laser.

51. The device according to claim 34, wherein the first determination unit is configured to:
obtain a first target image;
obtain a first template image, wherein the first template image contains the region positioning feature;
adjust, in response to a determination that the first target image is not matched with the first template image, a position of the measurement probe and/or the fixing portion to obtain a new first target image until the new first target image is matched with the first template image; and
determine a region corresponding to the measurement probe and/or the fixing portion as the measurement region in response to a determination that the first target image is matched with the first template image.

52. The device according to claim 51, further comprising a first image acquisition portion arranged on the measured object, the measurement probe, the fixing portion or other objects, wherein the first image acquisition portion is configured to acquire the first target image.

53. The device according to claim 34, wherein the first determination unit is configured to:
obtain a second target image, wherein the second target image contains the region positioning feature;
adjust, in response to a determination that a position of the region positioning feature in the second target image is not a first predetermined position, a position of the measurement probe and/or the fixing portion to obtain a new second target image until the position of the region positioning feature in the new second target image is the first predetermined position; and
determine a region corresponding to the measurement probe and/or the fixing portion as the measurement region in response to a determination that the position of the region positioning feature in the new second target image is the first predetermined position.

54. The device according to claim 53, further comprising a second image acquisition portion arranged on the measured object, the measurement probe, the fixing portion or other objects, wherein the second image acquisition portion is configured to acquire the second target image.

55. The device according to claim 54, wherein the region positioning feature is not arranged on the measurement probe when it is determined that the second image acquisition portion is arranged on the measurement probe; and
wherein the region positioning feature is not arranged on the fixing portion when it is determined that the second image acquisition portion is arranged on the fixing portion.

56. The device according to claim 34, wherein the first adjustment unit is configured to:
obtain a second projection feature;
adjust, in response to a determination that the first posture positioning feature is not matched with the second projection feature, the current measurement posture until the first posture positioning feature is matched with the second projection feature; and
determine that the current measurement posture is the target measurement posture, in response to a determination that the first posture positioning feature is matched with the second projection feature.

57. The device according to claim 56, further comprising a first posture positioning portion arranged on the measured object, the measurement probe, the fixing portion or other objects, wherein the first posture positioning portion is configured to project the second projection feature.

58. The device according to claim 57, wherein the first posture positioning feature is not arranged on the measurement probe when it is determined that the first posture positioning portion is arranged on the measurement probe; and
wherein the first posture positioning feature is not arranged on the fixing portion when it is determined that the first posture positioning portion is arranged on the fixing portion.

59. The device according to claim 57, wherein the first posture positioning portion comprises a second laser.

60. The device according to claim 34, wherein the first adjustment unit is configured to:
obtain a third target image;
obtain a second template image, wherein the second template image contains the first posture positioning feature;
adjust, in response to a determination that the third target image is not matched with the second template image, the current measurement posture to obtain a new third target image until the new third target image is matched with the second template image; and
determine that the current measurement posture is the target measurement posture, in response to a determination that the new third target image is matched with the second template image.

61. The device according to claim 60, further comprising a third image acquisition portion arranged on the measured object, the measurement probe, the fixing portion or other objects, wherein the third image acquisition portion is configured to acquire the third target image.

62. The device according to claim 34, wherein the first adjustment unit is configured to:
obtain a fourth target image, wherein the fourth target image contains the first posture positioning feature;
adjust, in response to a determination that a position of the first posture positioning feature in the fourth target image is not a second predetermined position, the current measurement posture to obtain a new fourth target image until the position of the first posture positioning feature in the new fourth target image is the second predetermined position; and
determine that the current measurement posture is the target measurement posture, in response to a determination that the position of the first posture positioning feature in the new fourth target image is the second predetermined position.

63. The device according to claim 62, further comprising a fourth image acquisition portion arranged on the measured object, the measurement probe, the fixing portion or other objects, wherein the fourth image acquisition portion is configured to acquire the fourth target image.

64. The device according to claim 63, wherein the first posture positioning feature is not arranged on the measurement probe when it is determined that the fourth image acquisition portion is arranged on the measurement probe; and
wherein the first posture positioning feature is not arranged on the fixing portion when it is determined that the fourth image acquisition portion is arranged on the fixing portion.

65. The device according to claim 34, further comprising:
a third determination module configured to determine a second posture positioning feature in response to the current measurement posture being not the target measurement posture, if the measurement probe is arranged at the position corresponding to the measurement region; and
an adjustment module configured to adjust the current measurement posture to the target measurement posture according to the second posture positioning feature.

66. The device according to claim 65, wherein the adjustment module comprises:
a first obtaining unit configured to obtain a third projection feature;
a second adjustment unit configured to adjust, in response to a determination that the second posture positioning feature is not matched with the third projection feature, the current measurement posture until the second posture positioning feature is matched with the third projection feature; and
a second determination unit configured to determine that the current measurement posture is the target measurement posture, in response to a determination that the second posture positioning feature is matched with the third projection feature.

67. The device according to claim 66, further comprising a second posture positioning portion arranged on the measured object, the measurement probe, the fixing portion or other objects, wherein the second posture positioning portion is configured to project the third projection feature.

68. The device according to claim 67, wherein the second posture positioning feature is not arranged on the measurement probe and the fixing portion when it is determined that the second posture positioning portion is arranged on the measurement probe; and
wherein the second posture positioning feature is not arranged on the measurement probe and the fixing portion when it is determined that the second posture positioning portion is arranged on the fixing portion.

69. The device according to claim 67, wherein the second posture positioning portion comprises a third laser.

70. The device according to claim 65, wherein the adjustment module comprises:
a second obtaining unit configured to obtain a fifth target image;
a third obtaining unit configured to obtain a third template image, wherein the third template image contains the second posture positioning feature;
a third adjustment unit configured to adjust, in response to a determination that the fifth target image is not matched with the third template image, the current measurement posture to obtain a new fifth target image until the new fifth target image is matched with the third template image; and
a third determination unit configured to determine that the current measurement posture is the target measurement posture, in response to a determination that the new fifth target image is matched with the third template image.

71. The device according to claim 70, further comprising a fifth image acquisition portion arranged on the measured object, the measurement probe, the fixing portion or other objects, wherein the fifth image acquisition portion is configured to acquire the fifth target image.

72. The device according to claim 65, wherein the adjustment module comprises:
a fourth obtaining unit configured to obtain a sixth target image, wherein the sixth target image contains the second posture positioning feature;
a fourth adjustment unit configured to adjust, in response to a determination that a position of the second posture positioning feature in the sixth target image is not a third predetermined position, the current measurement posture to obtain a new sixth target image until the position of the second posture positioning feature in the new sixth target image is the third predetermined position; and
a fourth determination unit configured to determine that the current measurement posture is the target measurement posture, in response to a determination that the position of the second posture positioning feature in the new sixth target image is the third predetermined position.

73. The device according to claim 72, further comprising a sixth image acquisition portion arranged on the measured object, the measurement probe, the fixing portion or other objects, wherein the sixth image acquisition portion is configured to acquire the sixth target image.

74. The device according to claim 73, wherein the second posture positioning feature is not arranged on the measurement probe and the fixing portion when it is determined that the sixth image acquisition portion is arranged on the measurement probe; and
wherein the second posture positioning feature is not arranged on the measurement probe and the fixing portion when it is determined that the sixth image acquisition portion is arranged on the fixing portion.

75. The device according to claim 65, further comprising:
a prompt module configured to generate a prompt information, wherein the prompt information is configured to prompt a completion of a positioning of the measurement posture and/or a positioning of the measurement region, and a form of the prompt information comprises at least one selected from an image, a speech, or a vibration.

76. The device according to claim 32, wherein the measurement module comprises a light source unit, an acquisition unit and a seventh determination unit;
the light source unit is configured to illuminate a measurement region by incident light having at least one predetermined wavelength, wherein each beam of the incident light is incident on an incident position to form at least one beam of exit light exited from at least one exit position on the measurement region;
the acquisition unit is configured to obtain a light intensity value corresponding to each beam of the exit light acquired by the measurement probe, so as to obtain T output light intensities, wherein the measurement probe comprises M photosensitive surfaces, and each of the T output light intensities is obtained by processing the light intensity value of the exit light acquired by one or more of the M photosensitive surfaces, 1≤T≤M; and
the seventh determination unit is configured to determine a concentration of a measured tissue component according to at least one output light intensity corresponding to the at least one predetermined wavelength.

77. The device according to claim 76, wherein the seventh determination unit is configured to:
determining, for each predetermined wavelength in the at least one predetermined wavelength, a first output light intensity and a second output light intensity from at least two output light intensities corresponding to the predetermined wavelength;
performing a differential processing on the first output light intensity and the second output light intensity corresponding to the predetermined wavelength to obtain a differential signal; and
determining the concentration of the measured tissue element according to the differential signal corresponding to each predetermined wavelength.

78. The device according to claim 76, wherein each photosensitive surface is configured to acquire the light intensity value of the exit light exited from an exit position within a predetermined anti-jitter range corresponding to the photosensitive surface.

79. The device according to claim 76, wherein a ratio of an average optical path of the exit light received by each photosensitive surface in a target tissue layer to a total optical path is greater than or equal to a ratio threshold, and the total optical path is a total distance that the exit light travels in the measurement region.

80. The device according to claim 76, wherein a total area of a homogeneous photosensitive surface is determined according to a tissue structure feature in the measurement region, the homogeneous photosensitive surface comprises one or more photosensitive surfaces, and the homogeneous photosensitive surface is configured to output one output light intensity.

81. The device according to claim 76, wherein a ratio of an area of each photosensitive surface to a circumference of the photosensitive surface is greater than or equal to a ratio threshold.

82. The device according to claim 81, wherein the ratio threshold is greater than or equal to 0.04 mm.

83. The device according to claim 78 or 79, wherein the photosensitive surface is in contact or non-contact with a surface of the measurement region.

84. The device according to claim 83, wherein a distance between the photosensitive surface and the surface of the measurement region is less than or equal to a distance threshold, and an efficiency of the photosensitive surface receiving the exit light is greater than or equal to an efficiency threshold.

85. A wearable apparatus, comprising the device of measuring the tissue element according to any one of claims 32 to 84.

86. The wearable apparatus according to claim 85, wherein a mass of the wearable apparatus is less than or equal to a mass threshold, so that a movement pattern of the wearable apparatus is consistent with a skin jitter pattern at the measurement region.

87. The wearable apparatus according to claim 85, wherein the wearable apparatus causes a movement amplitude of a skin at the measurement region to be less than or equal to a movement amplitude threshold.
